# EUROPEAN PATENT SPECIFICATION

(11) **EP 1 088 059 B1**
(45) Date of publication and mention of the grant of the patent: **15.09.2004**
(21) Application number: 99929106.5
(22) Date of filing: 23.06.1999
(51) Int. Cl.: C12N 9/96, C11D 3/386

(54) **A POLYPEPTIDE-POLYMER CONJUGATE**
POLYPEPTID-POLYMER KONJUGAT
CONJUGUE POLYPEPTIDE-POLYMERE

(30) Priority: 23.06.1998 DK 80998
(43) Date of publication of application: 04.04.2001
(73) Proprietor: Novozymes A/S, 2880 Bagsvaerd (DK)
(72) Inventor: DEUSSEN, Heinz-Josef, 2880 Bagsvaerd (DK); OLSEN, Arne Agerlin, 2880 Bagsvsaerd (DK); FATUM, Tine Muxoll, 2880 Bagsvaerd (DK); ROGGEN, Erwin Ludo, 2880 Bagsvaerd (DK)
(86) International application number: PCT/DK1999/000359
(87) International publication number: WO 1999/067370

(56) References cited:
- WO-A1-89/01033
- WO-A1-94/13311
- WO-A1-96/17929
- WO-A1-96/40792
- WO-A1-97/30148
- WO-A2-97/24421

## Description

### FIELD OF THE INVENTION

The present invention relates to a polypeptide-polymer conjugate wherein the polymer is a graft, block, alternate, or random co-polymer coupled to the surface of the polypeptide. The invention also relates to industrial compositions and products comprising a conjugate of the invention, the use of a polypeptide-polymer conjugate of the invention for reducing the allergenicity of industrial compositions and products, and finally a method for reducing the allergenicity of polypeptides.

### BACKGROUND OF THE INVENTION

For both medical and industrial applications the use of polypeptides, including enzymes, are well-known in the art. As polypeptides may potentially cause an undesired immune response - dependent on the way of challenge - typically an IgG and/or IgE response, techniques for reducing it have been developed during the last three decades.

One technique is the coupling technique where a number of polymeric molecules are coupled to the polypeptide in question. When using this technique the immune system have difficulties recognizing the epitopes (on the polypeptide's surface) responsible for the formation of antibodies, thereby reducing the immune response.

For polypeptides introduced directly into the circulatory system of the human body to give a particular physiological effect (*i.e.* pharmaceuticals) the typical potential immune response is an IgG and/or IgM response, while polypeptides which are inhaled through the respiratory system (*i.e.* industrial polypeptide) potentially may cause an IgE response (*i.e.* allergic response).

One of the theories explaining the reduced immune response is that the polymeric molecule(s) shield(s) epitope(s) on the surface of the polypeptide responsible for the immune response leading to antibody formation. Another theory or at least a partial factor is that the heavier the conjugate is the more reduced immune response is obtained.

Typically the polymers used for coupling to polypeptide to form conjugates are homopolymers, *i.e.* consisting of one repeating unit, *e.g*., ethylene oxide (EO), especially polyethylene glycol (PEG), or propylene oxide (PO), especially polypropylene glycol (PPG). Sacchareides, such as dextran have also been used.

US patent no. 4,179,337 concerns non-immunogenic polypeptides, such as enzymes and peptide hormones coupled to polyethylene glycol (PEG) or polypropylene glycol (PPG).

WO 96/17929 (Novo Nordisk A/S) concerns modified polypeptide conjugates suitable for industrial applications coupled to polymeric molecules; in particular polyethylene glycol (PEG) to reduce respiratory allergenicity.

### SUMMARY OF THE INVENTION

The present invention relates to a polypeptide-polymer conjugate suitable for industrial applications and incorporation as active ingredients in industrial products.

The present inventors have found that when coupling graft, block, alternate, or random co-polymers with the general formula:

EOₓPO_{y} (I)

wherein x=1-99%, y=1-99%, and x+y=100% covalently to a parent polypeptide, used for industrial application, the respiratory allergenicity is reduced when compared to the parent enzyme and even when compared to a corresponding conjugate coupled with PEG or other homopolymers.

In a second aspect the invention relates to compositions for use in industrial products comprising a conjugate of the invention.

In a third aspect the invention relates to a method for reducing the allergenicity of polypeptides.

### Industrial polypeptides

Polypeptides used for industrial applications often have an enzymatic and/or anti-microbial activity. Industrial polypeptides are (in contrast to pharmaceutical polypeptides) not intended to be introduced into the circulatory system of the body.

Therefore, it is not very likely that industrial polypeptides, such as enzymes, used as active ingredients in industrial compositions and/or products (defined below), such as detergents, such as laundry and dish washing detergens, food or feed addivites, including additives for bread making, composition for treating textiles, and personal care products, including cosmetics, come into direct contact with the circulatory system of the body of humans or animals, as such polypeptides (or products comprising such polypeptides) are not injected (or the like) into the bloodstream.

Thus, in the case of the industrial polypeptide the potential risk is respiratory allergy (i.e. IgE response) as a consequence of inhalation of polypeptides through the respiratory passage.

In the context of the present invention "industrial polypeptides" are defined as polypeptides, including peptides, proteins and/or enzymes, which are not intended to be introduced into the circulatory system of the body of humans and/or animals.

Examples of such polypeptide include polypeptides with enzymatic activity as defined below.

The invention also relates to industrial compositions and products having reduced respiratory allergenicity.

Furthermore, the invention relates to the use of a polypeptide-polymer conjugate of the invention for reducing the respiratory allergenicity of industrial composition and products.

Finally the invention relates to a method for reducing the allergenicity of polypeptides, in particular enzymes, by coupling one or more a graft, block, alternate, or random copolymer to a parent unmodified polypeptide.

### BRIEF DESCRIPTION OF THE DRAWINGS

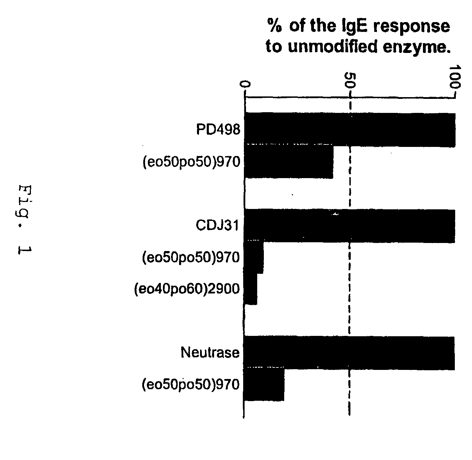

The figure shows the IgE response in Brown Norway rats sera to modified and unmodified enzymes.

### DETAILED DESCRIPTION OF THE INVENTION

The present invention relates to a polypeptide-polymer conjugate suitable for industrial applications and incorporation as active ingredients in industrial products. Conjugates of the invention have reduced respiratory allergenicity.

The term "polypeptide-polymer conjugate" means in the context of the present invention a polypeptide with one or more polymers covalently coupled thereto.

The term "reduced allergenicity" means in the context of the present invention that the amount of produced IgE (in humans, and molecules with comparable effects in specific animals), which can lead to an allergic state, is decreased when inhaling a modified polypeptide of the invention in comparison to the corresponding parent polypeptide. The term "respiratory allergenicity" may be used instead.

The present inventors have found that when a parent unmodified polypeptide is coupled to graft, block, alternate, or random co-polymers the potential allergenic response caused by inhalation of the polypeptide is reduced in comparison to a corresponding parent unmodified polypeptide and even in comparison to a corresponding conjugate wherein a corresponding number of homopolymers, such as PEG, have been coupled to the corresponding parent polypeptide.

It is well known, that a polymer can adopt different conformation/morphologies depending mainly, but not only on its molecular architecture, the solvent (here water), the temperature, and the concentration (S. Förster and M. Antonietti, Adv. Mater, 1998, 10, No.3, pp 195-217). Those conformation/morphologies include micelles of various shapes, lamellae, ordered cylinders, or bicontinous structures. The molecular conformation of co-polymers in aqueaous media like a solvated random coil, an extended coil, a rod-like polymer, a hypercoil, and a vesicle are well known (Water soluble polymers, M. J. Comstock Ed., ACS Symposium Series, 1991).

Thus, without being limited to any theory it is believed that a graft, block, alternate, or random co-polymer linked to the polypeptide surface adopts a conformation in water which yields to a better shielding of the surface as does a more hydrophilic homopolymer. Also synergistic effects due to the formation of supramolecular structures may reduce the accessibility of the polypeptide surface. Furthermore, an increased repulsion of the more lipophilic copolymer (in comparison to a PEG homopolymer) with the antibody might play a role.

Further, the more rigid structure (compared to homopolmers) of graft, block, alternate, or random co-polymer may make it more difficult for the antibody to "find its way" (through the more ridgid polymers and the adopted conformation) to the epitope on the polypeptide surface responsible for the IgE formation which results in an allergic response.

The hydrophobicity of the polymer is also believed to have an influence on the potential allergenicity of a polypeptide-polymer conjugate. Therefore, the more hydrophibic the polymer is the better does it shield the polypeptide.

By a proper choice of polymer and molecular architecture optimal coverage with respect shielding epitopes on the surface of the polypeptide can be obtained. Furthermore, by adjusting the properties of the attached polymers, optimized properties for different formulations, *e.g.* in fats or creams, can be obtained.

In the first aspect the invention relates to a polypeptide-polymer conjugate having coupled one or more polymers covalently to the parent polypeptide, wherein the polymer is characterized by the general formula:

EOₓPO_{y} (I)

wherein x=1-99%, y=1-99%, and x+y=100%.
"EO" means ethylene oxide and "PO" means propylene oxide.The molecular weight of the polymer typically lies in the range from 100 Da to 100,000 Da, preferably 100 to 50,000 Da, especially 100 to 10,000 Da.

In a preferred embodiment the polymer(s) comprise ethylene oxide units (EO) and propylene oxide units (PO) in a ration in the range from 10:90 or 20:80 or 30:70, 40:60, 50:50, 60:40, 70:30, 80:20 or 90:10.

### Assessment of allergenicity

Allergenicity may be assessed on the basis of inhalation tests, comparing the effect of intratracheally (into the trachea) administrated parent polypeptide with the corresponding modified polypeptide according to the invention.

A number of *in vivo* animal models exist for assessment of the allergenicity of polypeptide. Some of these models give a suitable basis for hazard assessment in man. Suitable models include a guinea pig model and a mouse model. These models seek to identify respiratory allergens as a function of elicitation reactions induced in previously sensitised animals. According to these models the alleged allergens are introduced intratracheally into the animals.

A suitable strain of guinea pigs, the Dunkin Hartley strain, do not as humans, produce IgE antibodies in connection with the allergic response. However, they produce another type of antibody the IgG1A and IgG1B (see *e.g*. Prentø, ATLA, 19, p. 8-14, 1991), which are responsible for their allergenic response to inhaled polypeptides including enzymes. Therefore, when using the Dunkin Hartley animal model, the relative amount of IgG1A and IgG1B is a measure of the allergenicity level.

A rat strain suitable for intratracheal exposure to polypeptides, such as enzymes, is the Brown Norway strain. Brown Norway rats produce IgE as the allergic response.

More details on assessing respiratory allergens in guinea pigs and mice is described by Kimber et al., (1996), Fundamental and Applied Toxicology, 33, p. 1-10.

Other animals such as *e.g*. rabbits may also be used for comparable studies.

### The polymeric molecule

The polymer coupled to the polypeptide is a graft, block, alternate, or random co-polymer having the general formula:

EOₓPO_{y} (I)

wherein x=1-99%, y=1-99%, and x+y=100%.

The polymer is preferably characterized by the general formula: (I) wherein x=10-90%, y=10-90%, and x+y=100%.

In a preferred embodiment of the invention the polymer consists of ethylene oxide units and propylene oxide units in a ration (EO unit: PO unit) of 10:90, 20:80, 30:70, 40:60, 50:50, 60:40, 70:30, 80:20, and 90:10.

In a preferred embodiment said polymer has a molecule weight from 100 to 100,000 Da, in particular 100 to 50,000 Da, especially 100 to 10,000 Da.

In a more preferred embodiment said polymer has a molecular weight from 100 to 12,000 Da, more preferred from 300 to 3,000 Da.

In an embodiment of the invention invention the polymer is a diblock, triblock, multiblock polymer. The general formula (I) should be interpreted as comprising polymers, wherein the EO units and PO units are placed independently.

Examples of specific block polymers which may be used to couple to the surface of the polypeptide are: poly(propylene glycol)-block-poly(ethyleneglycol)-block-poly(propylene glycol);poly(ethylene glycol)-block-poly(propylene glycol)-block-poly(ethylene glycol); poly(propylene glycol)-block-poly(ethylene glycol)-block-poly(propylene glycol)mono butyl ether; poly(ethylene glycol)-block-poly(propylene glycol)-block-poly(ethylene glycol)mono butyl ether; poly(propylene glycol)-block-poly(ethylene glycol)-block-poly(propylene glycol)mono methyl ether; poly(ethylene glycol)-block-poly(propylene glycol) - block-poly(ethylene glycol)mono methyl ether.

Preferred block polymers are block polymers having the general formula: H(-OCH₂CH₂-)ₓ[-OCH(CH₃)CH₂-]_{y}(-OCH₂CH₂-)_{z}OH, having the average molecule weight (Mₙ) of 1,100 and the ethylene glycol content of 10 wt%, Mₙ= 1,900 and 50 wt%, Mₙ= 2,000 and 10 wt%, Mₙ= 2,800 and 10 wt%, Mₙ=2,800 and 15 wt%,Mₙ= 2,900 and 40 wt%, Mₙ= 4,400 and 30 wt%, Mₙ= 5,800 and 30 wt%, Mₙ= 8,400 and 80 wt%.

Other preferred block polymers are block polymers having the general formula: H [-OCH (CH₃)CH₂-]ₓ(-OCH₂CH₂-)_{y}[-OCH (CH₃)CH₂-]_{z}OH, having the average molecule weight (Mₙ) of 2,000 and the ethylene glycol content of 50 wt%, Mₙ= 2,700 and 40 wt%, and Mₙ= 3,300 and 10 wt%.

Examples of specific block polymers are p7120: Pluronics, commercial available from BASF (Germany), Tergitol commercial available from Union Carbide (USA), Synperonic coomercial available from Fluka (Switzerland).

Examples of specific co-polymers which may be used to couple to the surface of the polypeptide are: poly(ethylene glycol-co-propylene glycol), especially poly(ethylene glycol-co-propylene glycol) having an an average molecule weight Mₙ of 2,500 and 75 wt% ethylene glycol and an average molecule weight Mₙ of 12,000 and 75 wt% ethylene glycol; poly(ethylene glycol-co-propylene glycol) mono butyl ether, especially poly(ehtylene glycol-co-propylene glycol)monobutyl ether having an Mₙ of 970 and 50 wt% ethylene glycol, an Mₙ of 1,700 and 50 wt% ethylene glycol and an Mₙ of 3,900 and 50 wt% ehtylene glycol; poly(ethylene glycol-co-propylene glycol) mono methyl ether.

Preferred polymers are non-toxic polymers composed of *e.g.* PEG and PPG co-polymers. Polymers requiring a relatively simple chemistry for its covalently coupling to attachment groups on the enzyme's surface are preferred.

The graft, block, alternate or radom co-polymers may be star-shaped or branched.

### Preparation of suitable polymers

Polymers to be atteched to the surface of the parent polypeptide may be prepared using standard techniques known in the art. Further, various polymers is commercially available from companies such as BASF (Germany), Union Carbide (USA), Aldrich etc.

### Activation of polymers

If the polymer to be conjugated with the polypeptide in question is not active it must be activated by the use of a suitable technique. It is also contemplated according to the invention to couple the block or co- polymer to the polypeptide through a linker. Suitable linkers are well-known to the skilled person.

Methods and chemistry for activation of polymeric molecules as well as for conjugation of polypeptides are intensively described in the literature.

Commonly used methods for activation of insoluble polymers include activation of functional groups with cyanogen bromide, periodate, glutaraldehyde, biepoxides, epichlorohydrin, divinylsulfone, carbodiimide, sulfonyl halides, trichlorotriazine etc. (see R.F. Taylor, (1991), "Protein immobilisation. Fundamental and applications", Marcel Dekker, N.Y.; S.S. Wong, (1992), "Chemistry of Protein Conjugation and Crosslinking", CRC Press, Boca Raton; G.T. Hermanson et al., (1993), "Immobilized Affinity Ligand Techniques", Academic Press, N.Y.). Some of the methods concern activation of insoluble polymers but are also applicable to activation of soluble polymers *e.g.* periodate, trichlorotriazine, sulfonylhalides, divinylsulfone, carbodiimide etc. The functional groups being amino, hydroxyl, thiol, carboxyl, aldehyde or sulfydryl on the polymer and the chosen attachment group on the protein must be considered in choosing the activation and conjugation chemistry which normally consist of i) activation of polymer, ii) conjugation, and iii) blocking of residual active groups.

In the following a number of suitable polymer activation methods will be described shortly. However, it is to be understood that also other methods may be used.

Coupling polymeric molecules to the free acid groups of polypeptides may be performed with the aid of diimide and for e-xample amino-PEG or hydrazino-PEG (Pollak et al., (1976), J. Am. Chem. Soc., 98, 289-291) or diazoacetate/amide (Wong et al., (1992), "Chemistry of Protein Conjugation and Crosslinking", CRC Press).

Coupling polymeric molecules to hydroxy groups are generally very difficult as it must be performed in water. Usually hydrolysis predominates over reaction with hydroxyl groups.

Coupling polymeric molecules to free sulfhydryl groups can be reached with special groups like maleimido or the *ortho*pyridyl disulfide. Also vinylsulfone (US patent no. 5,414,135, (1995), Snow et al.) has a preference for sulfhydryl groups but is not as selective as the other mentioned.

Accessible Arginine residues in the polypeptide chain may be targeted by groups comprising two vicinal carbonyl groups.

Techniques involving coupling electrophilically activated PEGs to the amino groups of Lysines may also be useful. Many of the usual leaving groups for alcohols give rise to an amine linkage. For instance, alkyl sulfonates, such as tresylates (Nilsson et al., (1984), Methods in Enzymology vol. 104, Jacoby, W. B., Ed., Academic Press: Orlando, p. 56-66; Nilsson et al., (1987), Methods in Enzymology vol. 135; Mosbach, K., Ed.; Academic Press: Orlando, pp. 65-79; Scouten et al., (1987), Methods in Enzymology vol. 135, Mosbach, K., Ed., Academic Press: Orlando, 1987; pp 79-84; Crossland et al., (1971), J. Amr. Chem. Soc. 1971, 93, pp. 4217-4219), mesylates (Harris, (1985), supra; Harris et al., (1984), J. Polym. Sci. Polym. Chem. Ed. 22, pp 341-352), aryl sulfonates like tosylates, and para-nitrobenzene sulfonates can be used.

Organic sulfonyl chlorides, *e.g.* Tresyl chloride, effectively converts hydroxy groups in a number of polymers, *e.g.* PEG, into good leaving groups (sulfonates) that, when reacted with nucleophiles like amino groups in polypeptides allow stable linkages to be formed between polymer and polypeptide. In addition to high conjugation yields, the reaction conditions are in general mild (neutral or slightly alkaline pH, to avoid denaturation and little or no disruption of activity), and satisfy the non-destructive requirements to the polypeptide.

Tosylate is more reactive than the mesylate but also more unstable decomposing into PEG, dioxane, and sulfonic acid (Zalipsky, (1995), Bioconjugate Chem., 6, 150-165). Epoxides may also been used for creating amine bonds but are much less reactive than the above mentioned groups.

Converting PEG into a chloroformate with phosgene gives rise to carbamate linkages to Lysines. This theme can be played in many variants substituting the chlorine with N-hydroxy succinimide (US patent no. 5,122,614, (1992); Zalipsky et al., (1992), Biotechnol. Appl. Biochem., 15, p. 100-114; Monfardini et al., (1995), Bioconjugate Chem., 6, 62-69, with imidazole (Allen et al., (1991), Carbohydr. Res., 213, pp 309-319), with para-nitrophenol, DMAP (EP 632 082 Al, (1993), Looze, Y.) etc. The derivatives are usually made by reacting the chloroformate with the desired leaving group. All these groups give rise to carbamate linkages to the peptide.

Furthermore, isocyanates and isothiocyanates may be employed yielding ureas and thioureas, respectively.

Amides may be obtained from PEG acids using the same leaving groups as mentioned above and cyclic imid thrones (US patent no. 5,349,001, (1994), Greenwald et al.). The reactivity of these compounds are very high but may make the hydrolysis to fast.

PEG succinate made from reaction with succinic anhydride can also be used. The hereby comprised ester group make the conjugate much more susceptible to hydrolysis (US patent no. 5,122,614, (1992), Zalipsky). This group may be activated with N-hydroxy succinimide.

Furthermore, a special linker can be introduced. The oldest being cyanuric chloride (Abuchowski et al., (1977), J. Biol. Chem., 252, 3578-3581; US patent no. 4,179,337, (1979), Davis et al.; Shafer et al., (1986), J. Polym. Sci. Polym. Chem. Ed., 24, 375-378.

Coupling of PEG to an aromatic amine followed by diazotation yields a very reactive diazonium salt which *in situ* can be reacted with a peptide. An amide linkage may also be obtained by reacting an azlactone derivative of PEG (US patent no. 5,321,095, (1994), Greenwald, R. B.) thus introducing an additional amide linkage.

As some peptides do not comprise many Lysines it may be advantageous to attach more than one PEG to the same Lysine. This can be done *e*.*g*. by the use of 1,3-diamino-2-propanol.

PEGs may also be attached to the amino-groups of the enzyme with carbamate linkages (WO 95/11924, Greenwald et al.). Lysine residues may also be used as the backbone.

### Position of the coupled block or co-polymer(s)

Virtually all ionized groups, such as the amino group of Lysine residues, are on the surface of the polypeptide molecule (see for instance Thomas E. Creighton, (1993), "Proteins", W.H. Freeman and Company, New York). Therefore, the number of readily accessible attachment groups (*i*.*e*. amino groups) on the poly-peptide's surface equals the number of Lysine residues in the primary structure of the polypeptide plus the N-terminus amino group.

According to the invention from 1 to. 100 polymers, preferably 4 to 50 polymeric molecules, 5 to 35 polymers are coupled to the parent polypeptide in question.

### The parent polypeptide

The modified polypeptides of the invention may be prepared on the basis of parent polypeptides, typically having a molecular weight in the range from 1 to 100 kDa, preferably from 15 to 60 kDa, using any suitable technique known in the art.

The term "parent" polypeptide is intended to indicate any uncoupled polypeptide (*i.e.* a polypeptide to be modified). The polypeptide may preferably be of microbial origin, such as bacterial, filamentous fungus or yeast origin.
The parent polypeptide may be a naturally-occurring (or wild-type) polypeptide or may be a variant thereof.

Preferred polypeptides are enzymes and polypeptides with anti-microbial activity. In a preferred embodiment the enzyme is an enzyme suitable for skin care compositions and products having a substantially enzymatic activity in the pH range used in the skin care product.

When choosing a parent polypeptide it is advantageous to use a polypeptide with the a high number of attachment groups.

Further, in a preferred embodiment of the invention the block or co-polymers are spread broadly over the surface of the parent polypeptide. For enzymes it is preferred that no block or co- polymers are coupled in the area close to the active site.

In the present context "spread broadly" means positioned so that the polymeric molecules coupled to the attachment groups of the polypeptide shields different parts of the polypeptide surface, preferable the whole or close to the whole surface area to make sure that the relevant epitope(s) being recognisable are shielded and hereby not recognised by the immune system's antibodies. It is believed that the surface area of interaction between the polypeptide and an antibody lies in the range about 500 Å² (26 x 19Å) (see Sheriff et al. (1987), Proc. Natl. Acad. Sci. USA, Vol. 84, p. 8075).

For enzymes it is preferred, to ensure a minimal loss of enzymatic activity, not to couple polymers in a close distance of the active site. Generally seen it is preferred that no polymers are attached within 5 Å, preferred 10 Å from the active site.

Further, polypeptides having coupled polymers at known epitope recognisable by the immune system or close to said epitope are also considered advantageous according to the invention. If the position of the epitope(s) is(are) unknown it is advantageous to coupled as many polymers to the attachment groups available on the surface of the polypeptide. It is preferred that said attachment groups are spread broadly over the surface of the polypeptide in a suitable distance from the active site.

Parent polypeptides fulfilling the above claims to the distribution of coupled polymers on the surface of the polypeptide are preferred according to the invention.

For enzymes especially enzymes having no or only very few polymers (i.e. 0 to 2) coupled within a distance of 0 to 5 Å, preferably 0 to 10 Å from the active site are preferred.

### The enzyme activity

The parent enzyme may have any activity known to be used in industrial composition and products as defined below. Contemplated enzyme activities include Oxidoreductases (E.C. 1, "Enzyme Nomenclature, (1992), Academic Press, Inc.), such as laccase and Superoxide dismutase (SOD); Hydrolases E.C. 3, including proteases, especially Serin proteases such as subtilisins, and lipolytic enzymes; Transferases, (E.C. 2), such as transglutaminases (TGases); Isomerases (E.C. 5), such as Protein disulfide Isomerases (PDI).

### Hydrolases

### Proteolytic enzymes

Contemplated proteolytic enzymes include proteases selected from the group of acidic aspartic proteases, cysteine proteases, serine proteases, such as subtilisins, or metallo proteases, with the above indicated properties (*i.e.* number of attachment groups, position of attachment groups etc.).

Specific examples of suitable parent proteases having a suitable number of attachment groups are indicated in Table 1 below:

**Table 1**

| Enzyme | Number of attachment groups | Molecular weight kDa | Reference |
|---|---|---|---|
| PD498 | 13 | 29 | Seq. ID No. 2 WO 93/24623 |
| Savinase® | 6 | 27 | von der Osten et al., (1993), Journal of Biotechnology, 28, p. 55+ |
| Proteinase K | 9 | 29 | Gunkel et al., (1989), Eur. J. Biochem, 179, p. 185-194 |
| Proteinase R | 5 | 29 | Samal et al, (1990), Mol. Microbiol, 4, p. 1789-1792 |
| Proteinase T | 14 | 29 | Samal et al., (1989), Gene, 85, p. 329-333 |
| Subtilisin DY | 13 | 27 | Betzel et al. (1993), Arch. Biophys, 302, no. 2, p. 499-502 |
| Lion Y | 15 | 46 | SEQ ID NO. 4 JP 04197182-A |
| Rennilase® | | 39 | Available from Novo Nordisk A/S |
| Ja16 | 5 | 28 | WO 92/17576 |
| Thermolysin | 12 | 34 | Titani et al., (1972) Nature New Biol. 238, p. 35-37, and SEQ ID NO 5 |
| Alcalase® (a natural subtilisin Carlberg variant) | 10 | 27 | von der Osten et al., (1993), Journal of Biotechnology, 28, p. 55+ |

The subtilisin PD498 has a molecular weight of 29 kDa, and as can be seen from SEQ ID NO: 2, 12 Lysine groups for polymer attachment on the surface of the enzyme plus one N-terminal amino group. As mentioned above preferred enzymes have Lysines spread broadly over the surface. PD498 has no Lysine residues in a distance of 0-10 Å from the active site which makes it especially suitable in modified form. Further, the Lysine residues are spread broadly on the surface of the enzyme (*i.e.* away from the active site).

The enzyme Subtilisin DY has a molecular weight of 27 kDa and has 12 amino groups (*i.e.* Lysine residues) on the surface of the enzyme and onc N-terminal amino group (see SEQ ID NO: 3).

The parent protease Lion Y has a molecular weight of 46 kDa and has 14 amino groups (*i.e.* Lysine residues) on the surface of the enzyme plus one N-terminal amino group (see SEQ ID NO: 4).

The neutral metallo protease Thermolysin has a molecular weight of about 34 kDa and has 11 amino groups (*i.e.* Lysine residues) on the surface plus one N-terminal amino group. (See SEQ ID NO: 5)

### Lipolytic enzymes

Contemplated lipolytic enzymes include include *Humicola lanuginosa* lipases, *e.g.* the one described in EP 258 068 and EP 305 216, *Humicola insolens,* a *Rhizomucor miehei* lipase, *e*.*g*. as described in EP 238 023, *Absidia* sp. lipolytic enzymes (WO 96/13578), a *Candida* lipase, such as a *C*. *antarctica* lipase, *e.g.* the *C*. *Antarctica* lipase A or B described in EP 214 761, a *Pseudomonas* lipase such as a *P*. *alcaligenes* and *P*. *pseudoalcaligenes* lipase, *e*.*g*. as described in EP 218 272, a *P*. *cepacia* lipase, *e.g.* as described in EP 331 376, a *Pseudomonas* sp. lipase as disclosed in WO 95/14783, a *Bacillus* lipase, *e.g.* a *B*. *subtilis* lipase (Dartois et al., (1993) Biochemica et Biophysica acta 1131, 253-260), a *B. stearothermophilus* lipase (JP 64/744992) and a *B*. *Pumilus* lipase (WO 91/16422). Other types of lipolytic include cutinases, e.g. derived from *Humicola insolens, Pseudomonas mendocina* (WO 88/09367), or *Fusarium solani pisi* (*e*.*g*. described in WO 90/09446).

### Oxidoreductases

### Laccases

Contemplated laccases include the laccases disclosed in WO 96/00290 and WO 95/33836 from Novo Nordisk.

Other oxidoreductases include catalase, glucose oxidase, peroxidase, haloperoxidase, superoxide dismutase, and lipoxygenase.

### Transferases

### Transglutaminases

Suitable transferases include any trnsglutaminases disclosed in WO 96/06931 (Novo Nordisk A/S) and WO 96/22366 (Novo Nordisk A/S).

### Isomerases

### Protein Disulfide Isomerase

Without being limited thereto suitable protein disulfide isomerases include PDIs described in WO 95/01425 (Novo Nordisk A/S).

### Industrial composition

In a further aspect of the invention relates to an "industrial composition" comprising a modified polypeptide with reduced allergenicity.

In the context of the present invention an "industrial composition" means a composition which is not intended to be introduced into the circulatory system. In other words it means a composition which is *not* intended for intradermally, intravenously.or subcutaneously administration.

As mentioned above the main problem for polypeptides, such as enzymes, for industrial application is the potential risk of respiratory allergy caused by inhalation through the respiratory system i.e. intratracheally or intranasal exposure.

Examples of "industrial composition" are polypeptides, especially enzymes and anti-microbial polypeptides, used in compositions or products such as detergents, including laundry and dish washing detergents, household article products, agro-chemicals, personal care products, such as skin care products, including cosmetics and toiletries, oral and dermal pharmaceuticals, compositions used for treating/processing textiles, compositions for hard surface cleaning, and compositions used for manufacturing food and feed, including food or feed additives, such as additives for making bread or the like etc. Especially contemplated according to the invention are skin care products and detergents.

### Skin Care Products

In the context of the present invention "skin care products" cover all personal care products used for cleansing, care and/or beautification of the skin of the body and further other products, such as hair care products, which during use may come in contact with the skin or respiratory system. Also corresponding products for animals are contemplated according to the present invention.

Specific examples of skin care products contemplated according to the present invention are soap, cosmetics, skin creams, skin gels, skin milk, skin lotion, cleansing cream, cleansing lotion, cleansing milk, cold cream, cream soap, makeup base, milky lotion, pack, calamine lotion, T zone essence, hand cream, essence powder, whitening powder, powder soap, cake soap, transparent soap, lip cream, lipstick, nourishing essence, creamy foundation, face powder, powder eye-shadow, powder foundation, nail polish remover, hair tonic, hair liquid, hair cream, hair gel, hair treatment, hair setting preparations, hair dyes, hair colorants, scalp treatment, shampoo, balsam, hair rinse, hair spray sun oil, sun screen, shaving foam and gel, shaving cream, baby oil, acne care products, antiperspirants, insect repellents, deodorants etc.

### Enzyme activities suitable for Skin Care

Skin care compositions of the invention comprise conjugates with reduced allergenicity of the invention and further ingredients known to be used in skin care compositions

A number of enzyme activities are known to be used skin care compositions.

### Proteases

Proteases are effective ingredients in skin cleaning products. Proteases remove the upper layer of dead keratinous skin cells and thereby makes the skin look brighter and more fresh. Further, proteases also improves the smoothness of the skin.

Proteases are used in toiletries, bath and shower products, including shampoos, conditioners, lotions, creams, soap bars, toilet soaps, and liquid soaps.

### Lipases

Lipases can be applied for cosmetic use as active ingredients in skin cleaning products and anti-acne products for removal of excessive skin lipids, and in bath and shower products such as creams and lotions as active ingredients for skin care.

Lipases can also be used in hair cleaning products (*e.g.* shampoos) for effective removal of sebum and other fatty material from the surface of hair.

### Oxidoreductases

The most common oxidoreductase for personal care purposes is an oxidase (usually glucose oxidase) with substrate (*e.g.* glucose) that ensures production of H₂O₂, which then will initiate the oxidation of for instance SCN⁻ or I⁻ into anti-microbial reagents (SCNO⁻ or I₂) by a peroxidase (usually lactoperoxidase). This enzymatic complex is known in nature from *e.g.* milk and saliva.

It is being utilised commercially as anti-microbial system in oral care products (mouth rinse, dentifrice, chewing gum) where it also can be combined with an amyloglucosidase to produce the glucose. These systems are also known in cosmetic products for preservation.

Another application of oxidoreductases are oxidative hair dyeing using oxidases, peroxidases and laccases (See *e*.*g*. WO 96/00290 or WO 95/33836 from Novo Nordisk).

Free radicals formed on the surface of the skin (and hair) known to be associated with the ageing process of the skin (spoilage of the hair).

The free radicals activate chain reactions that leads to destruction of fatty membranes, collagen, and cells.

The application of free radical scavengers such as Superoxide dismutase into cosmetics is well-known (R. L. Goldemberg, DCI, Nov. 93, p. 48-52).

Protein disulfide isomerase (PDI) is also an oxidoreductase. It may be utilised for waving of hair (reduction and reoxidation of disulfide bonds in hair) and repair of spoiled hair (where the damage is mainly reduction of existing disulfide bonds).

### Transglutaminase

Skin care compositions for application to human skin, hair or nails comprise (a) an amino-functional active ingredient, (b) transglutaminase to catalyse cross-linking of the active ingredient to the skin, hair or nails, and (c) a carrier is known from US patent no. 5,490,980.

A cosmetic composition suitable for application to mammalian skin, hair or nails comprising: (a) at least one corneocyte envelope protein in an amount sufficient to provide a protective layer on said skin, hair or nails; (b) a transglutaminase in an amount sufficient to form covalent bonds between the corneocyte envelope protein and externally exposed corneocyte proteins present in the stratum corneum of said skin, hair or nails; (c) calcium ions in an amount sufficient to activate the transglutaminase; and (d) a cosmetically acceptable vehicle, wherein the composition comprises an emulsion having two phases and wherein the corneocyte envelope protein is contained in one of the phases and the transglutaminase is contained within the other phase (see US patent no. 5,525,336).

JP 3083908 describes a skin cosmetic material contains a transglutaminase modified with a water-soluble substance. The modifying substance is, *e.g.*, one or more of polyethylene glycol, ethylene glycol, propylene glycol, glycerine, polyvinyl alcohol, glucose, sucrose, alginil acid, carboxymethyl cellulose, starch, and hydroxypropyl cellulose. The modification is done, *e.g.*, by introducing reactive groups and bonding to the enzyme. For providing a material mild to the skin, causing less time-lapse discolouring and odorising, and having good effects of curing rough skin, retaining moisture, and conditioning the skin beautifully.

### The Skin Care Products of the invention

In the third aspect the invention relates to a skin care product comprising a skin care composition of the invention. The term "skin care products" are defined above.

A skin care product of the invention may comprise from an effective amount of modified enzymes of the invention. Such effective amounts known to the skilled person may will often lie in the range from above 0 to 5% of the final skin care product..

Contemplated skin care products of the invention include, without being limited thereto, the following products: soap, cosmetics, skin creams, skin milk, skin lotion, skin gel, cleansing cream, cleansing lotion, cleansing milk, cold cream, cream soap, makeup base, milky lotion, pack, calamine lotion, T zone essence, hand cream, essence powder, whitening powder, powder soap, cake soap, transparent soap, lip cream, lipstick, nourishing essence, creamy foundation, face powder, powder eye-shadow, powder foundation, nail polish remover, hair tonic, hair liquid, hair cream, hair gel, hair treatment, hair setting preparations, hair dyes, hair colorants, scalp treatment, shampoo, balsam, hair rinse, hair spray sun oil, sun screen, shaving foam, shaving cream, baby oil, acne care products, antiperspirants, insect repellents, deodorants etc.

### General skin care product formulations

The term "ingredients used in skin care products" is meant to cover all ingredients which are known to be used in skin care product formulations. Examples of such ingredients ingredients can be found in "Cosmetics and Toiletries" edited by Wilfried Umbach and published by Ellis Horwood, Limited, England, (1991), and "Surfactants in Consumer Products", edited by J. Falbe and published by Spring-Verlag, (1987).

In the following a non exhausting list of guide formulations are listed. These provide an overwiev of formulations of important skin care products contemplated according to the invention.

| **Toilet soap** | | |
|---|---|---|
| Ingredients | Examples | % |
| Surfactants | Soap (sodium salt) | 83 -87 |
| Sequestering agents | Ethylenediamine tetraacetate | 0.1-0.3 |
| Consistency regulators 0.5 | Sodium chloride | approx. |
| Dyestuffs | | < 0.1 |
| Optical brighteners | | < 0.1 |
| Antioxidants | 2,6-bis(1,1-Dimethylethyl)-4-methyl phenol (BHT) | 0.1-0.3 |
| Whitening agents | Titanium dioxide | 0.1-0.3 |
| Fragrances | | 1.0-2.0 |
| Enzymes | Protease/Lipase | 0-5 |
| Water | Balance | |

| **Syndet (Synthetic Detergents)** | | |
|---|---|---|
| Ingredients | Examples | % |
| Surfactants | Lauryl sulfate | 30-50 |
| | Lauryl sulfo succinate | 1-12 |
| Refatting agents | Fatty alcohols | 10-20 |
| Plasticizers | Stearyl mono/diglycerides | 0-10 |
| Fillers | Starches | 0-10 |
| Active agents | Salicylic acid | 0-1 |
| Dyestuffs | | < 0.2 |
| Fragrances | | 0-2 |
| Enzymes | Protease/Lipase | 0-5 |
| Water | Balance | |

| **Foam bath and shower bath** | | | |
|---|---|---|---|
| Ingredients | Examples | % | % |
| Foam bath | Shower bath | | |
| Surfactants | Lauryl ether sulfate | 10-20 | 10-12 |
| | Coco amidopropyl dimethyl betaine | 2-4 | 2-4 |
| | Ethoxylated fatty acids | 0.5-2 | - |
| Refatting agents | Fatty alcohols | 0.5-3 | |
| | Ethoxylated fattyalcoho. | 0.5-5 | 0-4 |
| Enzymes | Protease/Lipase | 0-5 | 0-5 |
| | | | |

| Ingredients | Examples | % | % |
|---|---|---|---|
| Foam bath | Shower bath | | |
| Foam stabilizers | Fatty acid alkanol amide | 0.2-2 | 0-4 |
| Conditioners | Quaternized hydroxypropyl cellulose | - | 0-0.5 |
| Thickeners | Sodium chloride | 0-3 | 0-3 |
| Pearlescent agents | Ethyleneglycol stearate | 0-2 | - |
| Active agents | Vegetable extracts | 0-1 | 0-1 |
| Preservatives | 5-Bromo-5-nitro-1,3-dioxane | 0.1 | 0.1 |
| Dyestuffs | | 0.1-0.2 | 0.1 |
| Fragrances | | 0.3-3 | 0.3-2 |
| Enzymes | Protease/Lipase | 0-5 | 0-5 |
| Water | Balance | Balance | |

| **Skin cream (water-in-oil type and oil-in-water type)** | | | |
|---|---|---|---|
| Ingredients | Examples | % | |
| Water-in-oil/ Oil-in-water | | | |
| type Emulsifiers | type Sorbitane sesquioleate | 3-5 | - |
| Aluminum stearate | | 1-2 | - |
| Triethanolamine stearate | | - | 1-2 |
| Cetyl/Stearyl alcohol | | | |
| polyglycol ethers | | - | 1-3 |
| Fatty derivatives Isopropyl palmitate | | 1-5 | 0-3 |
| Cetyl/Stearyl alcohol | | - | 0-2 |
| 2-Octyl dodecanol | | 2-10 | 3-7 |
| Stearic/Palmitic acid | | - | 0-3 |
| Caprylic/Capric acid | | | |
| triglycerides | | 5-10 | - |
| Glycerine stearate | | - | 0-5 |
| Moisturizers | Glycerine | 1-5 | 1-5 |
| | Sorbitol | 1-5 | 1-5 |
| | Poly (hydroxy carboxylic acids) | 0.5-2 | - |
| | Propyleneglycol | - | 0-3 |
| Stabilizers | Magnesium sulfate | 0-0.8 - | |
| Preservatives | p-Hydroxy benzoic acid ester | 0.2- 0.4 | 0.2-0.4 |
| Enzymes | Protease/Lipase | 0-5 | 0-5 |
| Water | Balance | Balance | |

| **Body lotion (oil-in-water type) and skin lotion for application on the wet skin** | | | |
|---|---|---|---|
| Ingredients | Examples | % | % |
| Body lotion | Skin lotion | | |
| Emulsifiers | Cetyl/Stearyl alcohol | | |
| polyglycol ethers | | 1 -3 | - |
| Sorbitane monolaurate | | 0.5-1 - | |
| Sodium stearate | | - | 1-2 |
| Sodium lauryl ether sulfate - | | 0.5-2 | |
| Fatty derivatives 2-Octyl dodecanol | | 1-3 | 0-5 |
| Paraflin oils | | - | 20-25 |
| Bees wax | | 0.5-1 | - |
| Isooctyl stearate | | 3-7 - | |
| Isopropyl palmitate | | - | 2-5 |
| Moisturizers | Glycerine | 3-5 | 5-10 |
| Sorbitol | - | 0-5 | |
| Thickeners | Polyacrylates | 0-0.3 | 0-1 |
| | Methyl hydroxypropyl | 0-0.3 | 0-0.5 |
| | cellulose | | |
| Preservatives | p-Hydroxy benzoic acid ester | 0.2-0.4 | 0.2-0.4 |
| Enzymes | Protease/Lipase | 0-5 | 0-5 |
| Water | Balance | Balance | |

| **Face lotion** | | |
|---|---|---|
| Ingredients | Examples | % |
| Surfactants | Magnesium lauryl ether sulfate | 0.2-0.5 |
| Refatting agents | Di-n-butyl adipate | 1-2 |
| Solubilizers | Castor oil polyglycol ethers | 0.1-1 |
| Cleaning and refreshing components | Ethanol | 0-15 |
| Moisturizers | Glycerine | 0-5 |
| | Sorbitol | 0-5 |
| Preservatives | p-Hydroxy benzoic acid ester | 0.2-0.4 |
| Adstringents | Vegetable extracts | 1-5 |
| Antiirritants | Panthenol | 0-1 |
| | Allantoine | 0-0.2 |
| | Vegetable extracts | 0.5-3 |
| Enzymes | Protease/Lipase | 0-5 |
| Water | | Balance |

| **Hair shampoo** | | |
|---|---|---|
| Ingredients | Examples | % |
| Surfactants | Lauryl ether sulfate | 12-16 |
| | Coco fatty acid amidopropyl | 2-5 |
| | dimethyl betaine | |
| | Fatty acid polyglycol esters | 0-2 |
| Foam boosters | Fatty acid ethanol amides | 0.5-2.5 |
| Conditioners Protein hydrolysates | Quaternized hydroxyethyl cellulose | 0.4-1 0.2-1 |
| Refatting agents | Ethoxylated lanolin alcohols | 0.2-1 |
| Additives | Anti-dandruff agents | 0-1 |
| Preservatives | 5-Bromo-5-nitro-1,3-dioxane | 0.1-0.3 |
| Pearlescent agents Ethyleneglycol stearate | | 0-2 |
| Dyestuffs | | < 0.1 |
| pH-Regulators | Acids/Bases | 0.1-1 |
| Fragrances | | 0.3-0.5 |
| Enzymes | Protease/Lipase | 0-5 |
| Water | | Balance |

| **Hair rinse and hair conditioner** | | | |
|---|---|---|---|
| Ingredients | Examples | % | % |
| Hair rinse | Hair conditiner | | |
| Surfactants | Fatty alcohol poly- | | |
| | glycol ethers | 0.1-0.2 | 1.5-2.5 |
| | Cetyl trimethyl | | |
| | ammonium chloride | 0.5-1 | - |
| | Dimethyl benzyl | | |
| | stearyl ammonium chloride | - | 0.5-1 |
| Refatting agents | Cetyl/Stearyl mono/ diglyceride | 0.5-1.5 | 1.5-2.5 |
| Consistency regulators | Fatty alcohols | 1-2.5 | 2.5-3.5 |
| Thickeners | Methyl hydroxypropyl cellulose | 0.3-0.6 | 0.4-0.8 |
| Conditioners | Quaternized hydroxyethyl cellulose | 0.1-0.3 | 0.3-0.4 |
| Preservatives | p-Hydroxy benzoic acid ester | 0.1-0.3 | 0.1-0.3 |
| Dyestuffs | | <0.1 | <0.1 |
| pH-Regulators | Acids/Bases | 0,1-1 | 0.1-1 |
| Fragrances | | 0.2-0.5 | 0.2-0.5 |
| Enzymes | Protease/Lipase | 0-5 | 0-5 |
| Water | Balance | Balance | |

| **Hair dyes** | | |
|---|---|---|
| Ingredients | Examples | % |
| Component 1: | Alkaline dyeing cream | |
| Surfactants | Lauryl ether sulfate | 1-4 |
| | Ethoxylated castor oil | 1-2 |
| Consistency regulators | Fatty alcohols | 8-10 |
| Reductants | Sodium sulfite | 0.8-1.2 |
| Buffers | Ammonium chloride | 0.5-1 |
| Sequestrants | 1-Hydroxyethane-1,1-diphosphonic acid | 0.1-0.2 |
| Alkaline agents | Ammonia | 1.2-2 |
| Oxidation dyestuffsDeveloping agents 1 | | |
| Coupling agents | | 1 |
| Enzyme | Laccase | 0-5 |
| Water | | Balance |
| | | |

| Component II: | Hydrogen peroxide dispersion | |
|---|---|---|
| Surfactants | Lauryl ether sulfate | 0.5-1 |
| Oxidants | Hydrogen peroxide | 6-9 |
| Stabilizers | 1-Hydroxyethane-1,1-diphos phonic acid | 1-1.5 |
| Thickeners | Polyacrylates | 3-5 |
| Enzyme | Laccase | 0-5 |
| Water | | Balance |

| **Shaving cream** | | |
|---|---|---|
| Ingredients | Examples | % |
| Soaps | Palmitic/Stearic acid | 30-40 |
| | Potassium hydroxide | 5-7 |
| | Sodium hydroxide | 1-2 |
| Fatty components | Coconut oil | 5-10 |
| | Polyethyleneglycol | 0-2 |
| Stabilizers | Sodium tetraborate | 0-0.5 |
| | Sodium silicate | 0-0.5 |
| | Sorbitol | 0-3 |
| Enzyme | Protease | 0-5 |
| Water | | Balance |

| **Shaving lotion** | | |
|---|---|---|
| Ingredients | Examples | % |
| Disinfecting and phonic acid | Ethanol | 40-80 |
| Refatting agents | Di-n-butyl adipate | 1-2 |
| Solubilizers | Ethoxylated castor oil | 0.5-1 |
| Adstringents | Vegetable extracts | 1-10 |
| Antiirritants | Panthenol | 0-0.5 |
| Vegetable extracts | | 0-2 |
| Stabilizers | Glycerine | 0-5 |
| | Sorbitol | 0-5 |
| | Propyleneglycol | 0-3 |
| Enzymes | Protease | 0-5 |
| Water | | Balance |

| **Hair pomade** | | |
|---|---|---|
| Ingredients | Examples | % |
| Consistency regulators | Fatty alcohols | 4-5 |
| | Ethoxylated lanolin alcohols | 3-6 |
| Mineral fats | Vaseline | 45-52 |
| | Branched chain paraffins | 10-18 |
| Antioxidants | 2,6-bis(1,1-Dimethylethyl)-4-methyl phenol (BHT) | 0.5- 1 |
| Fragrances | | 0.2-0.4 |
| Dyestuffs | | 0.1 |
| Enzymes | Lipase | 0-5 |
| Emollients | Glycerine | Balance |

| **Setting lotion** | | |
|---|---|---|
| Ingredients | Examples | % |
| Solvents | Isopropanol | 12-20 |
| Film forming | Vinyl pyrrolidone/vinyl | |
| components | acetate copolymers | 2-3.5 |
| Softening agents | Vinyl pyrrolidone/dimethyl amino ethyl methacrylate | 0.2-1 |
| Conditioners | Protein hydrolysates | 0.2-0.5 |
| Antistatics | Cetyl trimethyl ammonium chloride | 0.1-0.5 |
| Emulsifiers | Etboxylated castor oil | 0.1-0.5 |
| Fragrances | | 0.1-0.2 |
| Dyestuffs | | < 0.1 |
| Enzymes | Lipase | 0-5 |
| Water | | Balance |

### Detergent disclosure

The detergent compositions of the invention may for example, be formulated as hand and machine laundry detergent compositions including laundry additive compositions and compositions suitable for use in the pretreatment of stained fabrics, rinse. added fabric softener compositions, and compositions for use in general household hard surface cleaning operations and dishwashing operations.

The detergent composition of the invention comprises the conjugate of the invention and a surfactant. Additionally, it may optionally comprise a builder, another enzyme, a suds suppresser, a softening agent, a dye-transfer inhibiting agent and other components conventionally used in detergents such as soil-suspending agents, soil-releasing agents, optical brighteners, abrasives, bactericides, tarnish inhibitors, coloring agents, and/or encapsulated or nonencapsula-ted perfumes.

The detergent composition according to the invention can be in liquid, paste, gels, bars or granular forms. The pH (measured in aqueous solution at use con-centration) will usually be neutral or alkaline, *e.g.* in the range of 7-11. Granular compositions according to the pre-sent invention can also be in "compact form", *i.e.* they may have a relatively higher density than conventional granular detergents, *i*.*e*. form 550 to 950 g/l. The enzyme conjugate of the invention, or optionally another enzyme incorporated in the detergent composition, is normally incorporated in the detergent composition at a level from 0.00001% to 2% of enzyme protein by weight of the composition, preferably at a level from 0.0001% to 1% of enzyme protein by weight of the composition, more preferably at a level from 0.001% to 0.5% of enzyme protein by weight of the composition, even more preferably at a level from 0.01% to 0.2% of enzyme protein by weight of the composition.

### Surfactant system

The surfactant system may comprise nonionic, anionic, cationic, ampholytic, and/or zwitterionic surfactants. The surfactant system preferably consists of anionic surfactant or a combination of anionic and nonionic surfactant, *e.g.* 50-100 % of anionic surfactant and 0-50 % nonionic. The laundry detergent compositions may also contain cationic, ampholytic, zwitterionic, and semi-polar surfactants, as well as the nonionic and/or anionic surfactants other than those already described herein.

The surfactant is typically present at a level from 0.1% to 60% by weight. Some examples of surfactants are described below.

### Nonionic surfactant

The surfactant may comprise polyalkylene oxide (e.g. polyethylene oxide) condensates of alkyl phenols. The alkyl group may contain from about 6 to about 14 carbon atoms, in a straight chain or branched-chain. The ethylene oxide may be present in an amount equal to from about 2 to about 25 moles per mole of alkyl phenol.

The surfactant may also comprise condensation products of primary and secondary aliphatic alcohols with about 1 to about 25 moles of ethylene oxide. The alkyl chain of the aliphatic alcohol can either be straight or branched, and generally contains from about 8 to about 22 carbon atoms.

Further, the nonionic surfactant may comprise polyethylene oxide conden-sates of alkyl phenols, condensation products of primary and secondary aliphatic alcohols with from about 1 to about 25 moles of ethylene oxide, alkylpolysaccharides, and mixtures hereof. Most preferred are C8-C14 alkyl phenol ethoxylates having from 3 to 15 ethoxy groups and C8-C18 alcohol ethoxylates (preferably C10 avg.) having from 2 to 10 ethoxy groups, and mixtures thereof.

### Anionic surfactants

Suitable anionic surfactants include alkyl alkoxyla-ted sulfates which are water soluble salts or acids of the formula RO(A)mSO3M wherein R is an unsubstituted C10-C-24 alkyl or hydroxyalkyl group having a C10-C24 alkyl com-ponent, preferably a C12-C20 alkyl or hydroxyalkyl, more pre-ferably C12-C18 alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (*e.g.*, sodium, potassium, lithium, calcium, magnesium, etc.), ammonium or substituted-ammonium cation. Alkyl ethoxy-lated sulfates as well as alkyl propoxylated sulfates are contemplated herein. Specific examples of substituted ammonium cations include methyl-, dimethyl, trimethyl-ammonium cations and quaternary ammo-nium cations such as tetramethyl-ammonium and dimethyl piperdinium cations and those derived from alkylamines such as ethylamine, diethylamine, triethyla-mine, mixtures thereof, and the like.

Other suitable anionic surfactants include the alkyl sulfate surfactants which are water soluble salts or acids of the formula ROS03M wherein R preferably is a C10-C24 hydrocarbyl, preferably an alkyl or hydroxyalkyl having a C10-C20 alkyl com-ponent, more preferably a C12-C18 alkyl or hydroxyalkyl, and M is H or a cation, *e.g*., an alkali metal cation (*e.g.* sodium, potassium, lithium), or ammonium or substituted ammonium.

Other anionic surfactants include salts (including, for example, sodium, potassium, ammonium, and substituted ammonium salts such as mono- di- and triethanola-mine salts) of soap, C8-C22 primary or secondary alkanesulfonates, C8-C24 olefinsulfonates, sulfonated polycarboxylic acids prepared by sulfonation of the pyrolyzed product of alkaline earth metal citrates. Alkylbenzene sulfonates are suitable, especially linear (straight-chain) alkyl benzene sulfonates (LAS) wherein the alkyl group preferably contains from 10 to 18 carbon atoms.

The laundry detergent compositions typically comprise from about 1% to about 40%, preferably from about 3% to about 20% by weight of such anionic surfactants.

### Builder system

The compositions according to the present invention may further comprise a builder system. Any conventional builder system is suitable for use herein including aluminosilicate materials, silicates, polycarboxylates and fatty acids, materials such as ethylenediamine tetraacetate (EDTA), metal ion sequestrants such as aminopolyph-osphonates. Phosphate builders can also be used herein.

Suitable builders can be an inorganic ion exchange material, commonly an in-organic hydrated aluminosilicate material, more particularly a hydrated synthetic zeo-lite such as hydrated zeolite A, X, B, HS or MAP.

Detergency builder salts are normally included in amounts of from 5% to 80% by weight of the composition. Preferred levels of builder for liquid detergents are from 5% to 30%.

### Other detergent enzyme activities

The detergent composition may, in addition to the conjugate of the invention with a specific activity, further comprise other enzyme activities *e.g.* also in the form of an enzyme conjugate as described according to the present invention, providing cleaning performance and/or fabric care benefits, *e.g.* proteases, lipases, cutinases, amylases, cellulases, peroxidases, haloperoxidases, oxidases (*e*.*g*. laccases).

Specific examples of contemplated enzymes are listed abobe in the section "The enzyme activity".

### Bleaching agents:

The detergent composition (especially in the case of a granular detergent) may also comprise a bleaching agents, *e.g.* an oxygen bleach or a halogen bleach. The oxyugen bleach may be a hydrogen peroxide releasing agent such as a perborate (e.g. PB1 or PB4) or a percarbonate, or it may e.g. be a percarboxylic acid. The parti-cle size may be 400-800 microns. When present, oxygen bleching compounds will typically be present at levels of from about 1% to about 25%.

The hydrogen peroxide releasing agent can be used in combination with bleach activators such as tetra-acetylethylenediamine (TAED), nonanoyloxybenzene-sulfonate (NOBS), 3,5-trimethyl-hexsanoloxybenzene-sulfonate (ISONOBS) or pen-taacetylglu-cose (PAG).

The halogen bleach may be, e.g. a hypohalite bleaching agent, for example, trichloro isocyanuric acid and the sodium and potassium dichloroisocyanurates and N-chloro and N-bromo alkane sulphonamides. Such materials are nor-mally added at 0.5-10% by weight of the finished product, preferably 1-5% by weight.

### Textile applications

### Proteases

Proteases are used for degumming and sand-washing of silk.

### Lipases

Lipases are used for removing fatty matter containing hy-drophobic esters (e.g. triglycerides) during the finishing of textiles (see e.g. WO 93/13256 from Novo Nordisk A/S).

### Oxidoreductases

In bleach clean-up of textiles catalases may serve to remove excess hydrogen peroxide.

### Carbohydrases

Cellulolytic enzymes are widely used in the finishing of denim garments in order to provide a localized variation in the co-lour density of the fabric (Enzyme facilitated "stone wash").

Also cellulolytic enzymes find use in the bio-polishing process. Bio-Polishing is a specific treatment of the yarn surface which improves fabric quality with respect to handle and appear-ance without loss of fabric wettability. Bio-polishing may be obtained by applying the method described *e.g.* in WO 93/20278.

During the weaving of textiles, the threads are exposed to con-siderable mechanical strain. In order to prevent breaking, they are usually reinforced by coating (sizing) with a gelatinous substance (size). The most common sizing agent is starch in native or modified form. A uniform and durable finishing can thus be obtained only after removal of the size from the fabric, the so called desizing. Desizing of fabrics sized with a size containing starch or modified starch is preferably fa-cili-tated by use of amylolytic enzymes.

### Food and Feed applications

Conjugated enzymes or polypeptides of the invention may advantageously be used in the manufactur-ing of food and feed.

### Proteases

The gluten in wheat flour is the essential ingredient responsible for the ability of flour to be used in baked foodstuffs. Proteolytic enzymes are sometimes needed to modify the gluten phase of the dough, e.g. a hard wheat flour can be softened with a protease.

Neutrase® is a commercially available neutral metallo protease that can be used to ensure a uniform dough quality and bread texture, and to improve flavour. The gluten proteins is degraded either moderately or more extensively to peptides, whereby close control is necessary in order to avoid excessive softening of the dough.

Proteases are also used for modifying milk protein.

To coagulate casein in milk when producing cheese proteases such as rennet or chymosin may be used.

In the brewery industry proteases are used for brewing with unmalted cereals and for controlling the nitrogen content.

In animal feed products proteases are used so to speak to expand the animals digestion system.

### Lipases

The application of lipase in the baking industry is rather new. Addition of lipase results in improved dough properties and an improved breadmaking quality in terms of larger volume, impro-ved crumb structure and whiter crumb colour. The observed ef-fect can be explained by a mechanism where the lipase changes the interaction between gluten and some lipids fragment during dough mixing. This results in an improved gluten network.

The flavour development of blue roan cheeses (e.g. Danablue), certain Italian cheese types and other dairy products containing butter fat are dependent on the degradation of milk fat into free fatty acids. Lipases may be used for developing flavour in such products.

In the oil- and fat producing industry lipases are used e.g. to minimize the amount of undesirable side-products, to modify fats by interesterification, and to synthesis of esters.

### Oxidoreductases

Further oxidoreductases with reduced allergenicity according to the invention may advantageously be used in the manufacturing of food and feed.

Several oxidoreductases are used for baking, glucose oxidase, lipoxygenase, peroxidase, catalase and combinations hereof. Traditionally, bakers strengthen gluten by adding ascorbic acid and potassium bromate. Some oxidoreductases can be used to replace bromate in dough systems by oxidation of free sulfydryl units in gluten proteins. Hereby disulphide linkages are formed resulting in stronger, more elastic doughs with greater resistance.

Gluzyme® (Novo Nordisk A/S) is a glucose oxidase preparation with catalase activity that can be used to replace bromate. The dough strengthen is measured as greater resistance to mechanical shock, better oven spring and larger loaf volume.

### Carbohydrases

Flour has varying content of amylases leading to differences in the baking quality. Addition of amylases can be necessary in order to standardize the flour. Amylases and pentosanases generally provide sugar for the yeast fermentation, improve the bread volume, retard retrogradation, and decrease the staling rate and stickiness that results from pentosan gums. Examples of carbohydrases is given below.

Certain maltogenic amylases can be used for prolonging the shelf life of bread for two or more days without causing gumminess in the product. Selectively modifies the gelatinized starch by cleaving from the non-reducing end of the starch molecules, low molecu-lar wight sugars and dextrins. The starch is modified in such a way that retrogradation is less likely to occur. The produced low-molecular-weight sugars improve the baked goods water retention capacity without creating the intermediate-length dextrins that result in gumminess in the finished product. The enzyme is inactivated during bread baking, so it can be considered a processing aid which does not have to be declared on the label. Overdosing of Novamyl can almost be excluded.

The bread volume can be improved by fungal α-amylases which further provide good and uniform structure of the bread crumb. Said α-amylases are endoenzymes that produce maltose, dextrins and glucose. Cereal and some bacterial α-amylases are inactivated at temperatures above the gelatinization temperature of starch, therefore when added to a wheat dough it results in a low bread volume and a sticky bread interior. Fungamyl has the advantage of being thermolabile and is inactivated just below the gelatinization temperature.

Enzyme preparations containing a number of pentosanase and hemi-cellulase activities can improve the handling and stability of the dough, and improves the fresh-ness, the crumb structure and the volume of the bread.

By hydrolysing the pentosans fraction in flour, it will lose a great deal of its water-binding capacity, and the water will then be available for starch and gluten. The gluten becomes more pliable and extensible, and the starch gelatinize more easily. Pentosanases can be used in combination with or as an alternative to emulsifi-ers.

Further carbohydrases are user for producing syrups from starch, which are widely used in soft drinks, sweets, meat products, dairy products, bread products, ice cream, baby food, jam etc.

The conversion of starch is normally carried out three steps.

First the starch is liquefied, by the use of α-amylases. Maltodextrins, primary consisting of oligosaccharides and dextrins, are obtained.

The mixture is then treated with an amyloglucosidase for hydrolysing the oligosaccharides and dextrins into glucose. This way a sweeter product is obtained. If high maltose syrups are desired b-amylases alone or in combina-tion with a pullulanase (de-branching enzyme) may be used.

The glucose mixture can be made even sweeter by isomerization to fructose. For this an immobilized glucose isomerase can be used.

In the sugar industry, it is common practice to speed up the break down of present starch in cane juices. Thereby the starch content in the raw sugar is reduced and filtration at the refinery facilitated.

Furthermore dextranases are used to break down dextran in raw sugar juices and syrups.

In the alcohol industry a-amylases is advantageously being used for thinning of starch in distilling mashes.

In the brewing industry a-amylases is used for adjunct liquefaction.

In the dairy industry b-galactosidases (lactase) is used when producing low lactose milk for persons suffering from lactose malabsorption.

When flavoured milk drinks are produced from lactase-treated milk, the addition of sugar can be reduced without reducing the sweetness of the product.

In the production of condensed milk, lactose crystallization can be avoided by lactase treatment, and the risk of thickening caused by casein coagulation in lactose crystals is thus reduced.

When producing ice cream made from lactase-treated milk (or whey) no lactose crystals will be formed and the defect, sandiness, will not occur.

Further, xylanases are known to be used within a number of food/feed industrial applications as described in WO 94/21785 (Novo Nordisk A/S).

α-amylases are used in the animal feed industry to be added to cereal-containing feed to improve the digestibility of starch.

### Anti-microbial polypeptides

Certain bacteriolytic enzymes may be used *e.g*. to wash carcasses in the meat packing industry (see US patent no. 5,354,681 from Novo Industri A/S).

### Transferases

Transglutaminases with reduced allergenicity according to the invention may advantageously be used in the manufacturing of food and feed.

Transglutaminases has the ability to crosslinking protein.

This property can be used for gelling of aqueous phases containing proteins. This may be used for when producing of spreads (wo 96/08156 from Novo Nordisk A/S).

Transglutaminases are being used for improve-ment of baking quality of flour *e.g.* by modifying wheat flour to be used in the pre-paration of cakes with improved properties, such as improved taste, dent, mouth-feel and a higher volume (see JP 1-110147).

Further producing paste type food material e.g. used as fat substitution in foods as ice cream, toppings, frozen desserts, mayonnaises and low fat spreads (see WO 93/22930 from Novo Nordisk A/S).

Furthermore for preparation of gels for yoghurt, mousses, chee-se, puddings, orange juice, from milk and milk-like products, and binding of chopped meat pro-duct, improve-ment of taste and texture of food proteins (see WO 94/21120 and WO 94/21129 from Novo Nordisk A/S).

### Phytases

Phytases of the invention may advantageously be used in the manufacturing of food, such as breakfast cereal, cake, sweets, drink, bread or soup etc., and animal feed.

Phytases may be used eit-her for exploiting the phosphorus bound in the phytate/phytic acid present in vegetable protein sources or for exploiting the nutritionally important minerals bound in phy-tic acid com-plexes.

Microbial phytase may be added to fe-ed-stuff of monogastric animals in or-der to avoid supplementing the feed with inorganic phosphorus (see US patent no. 3,297,548)

Further phytases may be used in soy processing. Soya-bean meal may con-tain high levels of the anti-nutritional factor phytate which renders this protein source unsuitable for application in baby food and feed for fish, calves and other non-ruminants, since the phytate chelates essential minerals present therein (see EP 0 420 358).

Also for baking purposes phytases may be used. Bread with better quality can be prepared by baking divided pieces of a dough containing wheat flour etc. and phytase (see JP-0-3076529-A).

A high phytase activity koji mold are known to be used for producing refined sake (see JP-0-6070749-A).

In a further aspect the invention relates to a the use of modified polypeptide of the invention for reducing the allergenicity of industrial compositions and products as defined above.

### MATERIAL AND METHODS

### Materials

### Materials

### Enzymes:

Neutrase®: Protease derived from *Bacillus amyloliquefaciens*. The sequence of Neutrase® is shown in SEQ ID NO: 6
PD498: Protease of subtilisin type shown in WO 93/24623. The sequence of PD498 is shown in SEQ ID NO: 1 and 2.
Subtilisin DY: Protease of the subtilisin type shown in SEQ ID NO: 3 isolated from *Bacillus* sp. variant (Betzel et al. (1993), Archives of Biophysics, Vol. 302, No. 2, p. 499-502).

### ELISA reagents:

Horse Radish Peroxidase labelled pig anti-rabbit-Ig (Dako, DK, P217, dilution 1:1000).
Rat anti-mouse IgE (Serotec MCA419; dilution 1:100). Mouse anti-rat IgE (Serotec MCA193; dilution 1:200).
Biotin-labelled mouse anti-rat IgGl monoclonal antibody (Zymed 03-9140; dilution 1:1000)
Biotin-labelled rat anti-mouse IgG1 monoclonal antibody (Serotec MCA336B; dilution 1:2000)
Streptavidin-horse radish peroxidase (Kirkegård & Perry 14-30-00; dilution 1:1000).

### Buffers and Solutions:

- PBS (pH 7.2 (1 liter))

| | |
|---|---|
| NaCl | 8.00 g |
| KCl | 0.20 g |
| K2HPO4 | 1.04 g |
| KH2PO4 | 0.32 g |

- Washing buffer PBS, 0.05% (v/v) Tween 20
- Blocking buffer PBS, 2% (wt/v) Skim Milk powder
- Dilution buffer PBS, 0.05% (v/v) Tween 20, 0.5% (wt/v) Skim Milk powder
- Citrate buffer (0.1M, pH 5.0-5.2 (1 liter))

| | |
|---|---|
| NaCitrate | 20.60 g |
| Citric acid | 6.30 g |

- Stop-solution (DMG-buffer)
- Sodium Borate, borax (Sigma)
- 3,3-Dimethyl glutaric acid (Sigma)
- CaCl₂ (Sigma)
- Tween 20: Poly oxyethylene sorbitan mono laurate (Merck cat no. 822184)
- N-Hydroxy succinimide (Fluka art. 56480))
- Phosgene (Fluka art. 79380)
- Lactose (Merck 7656)
- PMSF (phenyl methyl sulfonyl flouride) from Sigma
- Succinyl-Alanine-Alanine-Proline-Phenylalanine-paranitroanilide (Suc-AAPF-pNP) Sigma no. S-7388, Mw 624.6 g/mole.
- mPEG (Fluka)

### Colouring substrate:

OPD: o-phenylene-diamine, (Kementec cat no. 4260)

### Test Animals:

Brown Norway rats (from Charles River, DE)

### Equipment:

XCEL II (Novex)
ELISA reader (UVmax, Molecular Devices)
HPLC (Waters)
PFLC (Pharmacia)
Superdex-75 column, Mono-Q, Mono S from Pharmacia, SW.
SLT: Fotometer from SLT Lablnstruments
Size-exclusion chromatograph (Spherogel TSK-G2000 SW).
Size-exclusion chromatograph (Superdex 200, Pharmacia, SW)
Amicon Cell

### Methods:

### Intratracheal (IT) stimulation of Brown Norway rats

For IT administration of molecules disposable syringes with a 2½" long metal probe is used. This probe is instilled in the trachea of the rats approximately 1 cm below the epiglottis, and 0.1 ml of a solution of the molecules is deposited.

The test animals are Brown Norway rats (BN) in groups of 10. Weight at time of start is more than 200 grams and at termination approximately 450 grams.

### ELISA procedure to determine relative concentrations of IgE antibodies in Brown Norway rats.

A three layer sandwich ELISA is used to determine relative concentrations of specific IgE serum anti-bodies.
1) Coat the ELISA-plate with 10 mg mouse anti-rat IgE Buffer 1 (50microL/well). Incubate over night at 4°C.
2) Empty the plates and block with Blocking buffer for at least ½ hour at room temperature (200 microL/well). Shake gently. Wash the plates 3 times with Washing Buffer.
3) Incubate with rat sera (50 microL/well), starting from undiluted and continue with 2-fold dilutions. Keep some wells free for buffer 4 only (blanks). Incubate for 30 minutes at room temperature. Shake gently. Wash the plates 3 times in Washing Buffer.
4) Dilute the enzyme in Dilution buffer to the appropriate protein concentration. Incubate 50 microL/well for 30 minutes at room temperature. Shake gently. Wash the plates 3 times in Washing Buffer.
5) Dilute specific polyclonal anti-enzyme antiserum serum (pIg) for detecting bound antibody in Dilution buffer. Incubate 50 microl/well for 30 minutes at room temperature. Shake gently. Wash the plates 3 times in Washing Buffer.
6) Dilute Horseradish Peroxidase-conjugated anti-pIg-antibody in Dilution buffer. Incubate 50 microL/well at room temperature for 30 minutes. Shake gently. Wash the plates 3 times in Washing Buffer.
7) Mix 0.6 mg ODP/ml + 0.4 microL H₂O₂/ml in substrate Buffer. Make the solution just before use. Incubate for 10 minutes. 50 microL/well.
8) To stop the reaction, add 50 microL Stop Solution/well.
9 ) Read the plates at 492 nm with 620 nm as reference. Data is calculated and presented in Lotus.

### Determination of the molecular weight

Electrophoretic separation of proteins was performed by stan-dard methods using 4-20% gradient SDS polyacrylamide gels (Novex). Proteins were detected by silver staining. The molecular weight was measured relatively to the mobility of Mark-12® wide range molecular weight standards from Novex.

### Protease activity

### Analysis with Suc-Ala-Ala-Pro-Phe-pNa:

Proteases cleave the bond between the peptide and p-nitroaniline to give a visible yellow colour absorbing at 405 nm.

Buffer: *e.g*. Britton and Robinson buffer pH 8.3
Substrate: 100 mg suc-AAPF-pNa is dissolved into 1 ml dimethyl sulfoxide (DMSO). 100 ml of this is diluted into 10 ml with Britton and Robinson buffer.

### Analysis

The substrate and protease solution is mixed and the absorbance is monitored at 405 nm as a function of time and ABS_{405 nm}/min. The temperature should be controlled (20-50°C depending on protease). This is a measure of the protease activity in the sample.

### EXAMPLES

### Example 1

### Activation of poly(ethylene glycol)-block-poly(propylene glycol)-block-poly(ethylene glycol) 1.900 (50 wt% ehtyleneglycol) with N-succinimidyl carbonate

Poly(ethylene glycol)-block-poly(propylene glycol)-block-poly(ethylene glycol) 1.900 (50 wt% ehtyleneglycol)from ALDRICH was dissolved in toluene (5 ml/g of polymer). About 20% was distilled off at normal pressure to dry the reactants azeotropically. The solution was cooled to 20°C and phosgene in toluene (1.93 M, 7 mole/mole polymer) was added. The mixture was then stirred at room temperature overnight. The solvent and excess phosgene was removed in vacuo and the intermediate bis(chloroformate) was obtained as an oil.

Toluene (dry 4 ml/g polymer) was added to redissolve the oil. N-Hydroxy succinimide (NHS) (2.4 mole/mole polymer) was added and the mixture was cooled with an ice-bath. Triethylamine (2.2 mole/mole polymer) was added dropwise at 0°C. Immediate precipitation of triethylamine hydrochloride (Et3N.HCl) could be observed. The mixture was stirred overnight at room temperature. The mixture was filtered using a glass frit (G5) to remove the Et3N.HCl. The filtrate was evaporated to dryness under reduced pressure to yield 97 % (mole/mole) of an oil. NMR Indicating > 90% activation and <8 o/o (mole/mole) of unbound NHS. 1H-NMR (400MHz) for poly(ethylene glycol)-block-poly(propylene glycol)-block-poly(ethylene glycol) 1.900 bis(succinimidyl carbonate) (50 wt% ehtyleneglycol)(CDCl3) δ: 1.15 bs (I=330 -CH3 in PPG), 2.69 s (I=1.7 unreacted NHS), 2.83 s (I= 41, succinimide), 3.41 m (I=110, CH-CH2 in PPG), 3.55 m (I=220, CH-CH2 in PPG), 3.61 m (I=440 main peak), 4.46 t (I=19, CH2-O-CO- in PEG).

### Example 2

### Activation of poly(ethylene glycol)-block-poly(propylene glycol)-block-poly(ethylene glycol) 2.900 (ca. 40 wt% ethyleneglycol) with N-succinimidyl carbonate

Poly(ethylene glycol)-*block*-poly(propylene glycol)-*block*-poly(ethylene glycol) 2.900 (ca. 40 wt% ehtyleneglycol)from ALDRICH was dissolved in toluene (4.4 ml/g of polymer). About 15% was distilled off at normal pressure to dry the reactants azeotropically. The solution was cooled to 0°C and phosgene in toluene (1.93 M, 7 mole/mole polymer) was added. The mixture was then stirred at room temperature for 19 hours. The solvent and excess phosgene were removed *in vacuo* and the intermediate bis(chloroformate) was obtained as an oil.

Toluene (dry 2.5 ml/g polymer) was added to redissolve the oil. *N*-Hydroxy succinimide (NHS) (2.4 mole/mole polymer) was added at room temperature. Triethylamine (2.2 mole/mole polymer) was added dropwise. Immediate precipitation of triethylamine hydrochloride (Et₃N.HCl) could be observed. The mixture was stirred for 21 hours at room temperature. The mixture was then filtered using a glass frit (G5) to remove insoluble Et₃N.HCl. The filtrate was evaporated to dryness under reduced pressure to yield 96 % (mole/mole) of an oil. NMR Indicating > 70% activation and <26 o/o (mole/mole) of unbound NHS. ¹H-NMR (400MHz) for poly(ethylene glycol)-*block*-poly(propylene glycol)-block-poly(ethylene glycol) 2.900 bis(succinimidyl carbonate) (ca.40 wt% ethyleneglycol) (400 MHz, CDCl₃) δ: 1.15 bs (I=1000 - C**H**₃ in propylene glycol), 2.68 s (I=10.6 unreacted NHS), 2.84 s (I= 61.7, succinimide), 3.40 m (I=318, -C**H**-CH₂ in propylene glycol), 3.55 m (I=668, -CH-C**H**₂- in propylene glycol), 3.61 m (I=1022 main peak, -C**H**₂-C**H**₂- in ethylene glycol), 4.46 t (I=25.7, -C**H**₂-O-CO-).

### Example 3

### Activation of poly(ethylene glycol)-co-(propylene glycol) monobutyl ether 970 (ca. 50 wt% ethyleneglycol) with N-succinimidyl carbonate

Poly(ethylene glycol)-co-(propylene glycol) monobutyl ether 970 (ca. 50 wt% ethyleneglycol) from ALDRICH was dissolved in toluene (4 ml/g of polymer). About 25% was distilled off at normal pressure to dry the reactants azeotropically. The solution was cooled to 0°C and phosgene in toluene (1.93 M, 5 mole/mole polymer) was added. The mixture was then stirred at room temperature for 21 hours. The solvent and excess phosgene were removed *in vacuo* and the intermediate chloroformate was obtained as an oil.

Toluene (dry 2 ml/g polymer) was added to redissolve the oil. *N*-Hydroxy succinimide (NHS) (1.2 mole/mole polymer) was added at room temperature. Triethylamine (1.1 mole/mole polymer) was added dropwise at 0°C. Immediate precipitation of triethylamine hydrochloride (Et₃N.HCl) could be observed. The mixture was stirred overnight at room temperature. The mixture was then filtered using a glass frit (G5) to remove insoluble Et₃N.HCl. The filtrate was evaporated to dryness under reduced pressure to yield 89 % (mole/mole) of an oil. NMR Indicating > 72% activation and <5 o/o (mole/mole) of unbound NHS. ¹H-NMR (400MHz) for poly(ethylene glycol)-co-(propylene glycol) monobutyl ether 970 succinimidyl corbonate (ca. 50 wt% ethyleneglycol) 400 MHz, CDCl₃) δ: 0.91 t (I=1000 -C**H**₃ butyl), 1.15 bs (I=8744 -C**H**₃ in propylene glycol), 1.39 m (I=1320 CH₃-C**H**₂-C**H**₂- butyl), 1.55 m (I=656 -C**H**₂-O- butyl), 2.68 s (I=60.8 unreacted NHS), 2.83 s (I= 963.2, succinimide), 3.40 m (I=3059, C**H**-CH₂ in propylene glycol), 3.55 m (I=2678, CH-C**H**₂ in propylene glycol), 3.61 m (I=1764 main peak, -C**H**₂-C**H**₂- in ethylene glycol), 4.46 m (C**H**₂-O-CO-).

### Example 4

### Evaluation of the allergenic potencies of proteases modified with co-and blockpolymers

Each sample was diluted to 0.015 mg protein/ml, and aliquoted in 1.5 ml. These fractions was stored at -20 °C intil use. Additionally, 100 µl of the respective factions was stored in the lab-freezer at -20°C for immunochemical analysis at the beginning, halfway and at the end of the study. For each immunization and each analysis a new fraction was taken.

Twenty intratracheal immunizations was performed weekly with 100 µl of the protein dilution mentioned before. Thus, group 1 received unmodified PD498, group 2 PD498-EO₅₀PO₅₀970, group 3 unmodified Subtilisin DY (CDJ31), group 4 Subtilisin DY (CDJ31)-EO₅₀PO₅₀970, group 5 Sutilisin DY (CDJ31)-EO₄₀PO₆₀2900, group 6 Neutrase and group 7 Neutrase-EO₅₀PO₅₀970. Each group contained 10 rats. Control rats received 100 µl 0.9% NaCl. Blood samples (2 ml) was collected from the eye one week after every second immunization. Serum was obtained by blood clothing, and centrifugation.

Specific IgE levels was determined using the ELISA procedure. The figure shows that the IgE response is reduced for the modified enzymes compared to unmodified enzyme.

## Claims

1. A polypeptide-polymer conjugate suitable for industrial applications having coupled one or more polymers covalently to the parent polypeptide, wherein the polymer is **characterized by** the general formula:
EOₓPO_{y} (I)
wherein x=1-99%, y=1-99%, and x+y=100%.

2. The conjugate according to claim 1, wherein x=10-90%, y=10-90%, and x+y=100%.

3. The conjugate according to claims 1 or 2, wherein the polymer has a molecule weight from 100 to 100,000 Da, in particular 100 to 50,000 Da, especially 100 to 10,000 Da.

4. The conjugate according to claim 1 to 3, wherein the parent polypeptide has a molecule weight from 1 to 100 kDa, in particular an enzyme, has a molecular weight from 15 to 60 kDa.

5. The cunjugate according to any of claims 1 to 4, wherein the block or co-polymer(s) comprise ethylene oxide units (EO) and propylene oxide units (PO) in a ration in the range from 10:90 or 20:80 or 30:70, 40:60, 50:50, 60:40, 70:30, 80:20 or 90:10.

6. The conjugate according to any of claims 1 to 5, wherein from 1 to 100 polymeric molecules, preferably 4 to 50, especially 5 to 35 polymeric molecules, are coupled covalently to the parent enzyme.

7. The conjugate according to any of claims 1 to 6, wherein the polypeptide is of microbial origin, such as bacterial, filamentous fungus or yeast origin.

8. The conjugate according to any of claims 1 to 7, wherein the polypeptide is an enzyme from the group of hydrolase, including proteases, including serine proteases, such as subtilisins and metallo proteases, or lipases, or oxidoreductases, such as a laccase and haloperoxidases, or Superoxide dismutase.

9. The conjugate according to claim 8, wherein the parent protease is selected from the group including PD498, Savinase®, ProteinaseK, ProteinaseR Thermitase, Subtilisin DY, Lion Y, Alcalase®, ProteinaseT, Neutrase® and JA16.

10. The conjugate according to claim 9, wherein the enzyme is PD498 shown in SEQ ID NO: 1, or the subtilisin type protease Subtilisin DY shown in SEQ ID NO: 3, or Lion Y shown in SEQ ID NO: 4, or the metallo protease Thermolysin shown in SEQ ID NO: 5, or Neutrase® shown in SEQ ID NO: 6.

11. An industrial composition comprising a conjugate according to claim 1-10.

12. The industrial composition according to claim 11, wherein the composition comprises ingredients known to be used in personal care compositions, especially skin care compositions.

13. The industrial composition according to claims 12, being a detergent, such as a laundry detergent composition, dishwahs composition, or hard surface cleaning composition.

14. The industrial composition according to claims 11, being a composition, such as a food or feed addivtive, especially an additive for making bread or the like.

15. The industrial composition according to claims 11, being a composition for treating textiles.

16. The use of a conjugate of any of claims 1-10 for reducing the respiratory allergenicity of industrial compositions according to any of claims 11-16.

17. A method for reducing theallergenicity of polypeptide comprising coupling block or co-polymers as defined in any of claims 1-3, 5, 6 to a parent polypeptide of any claims 4, 7-10.

## Patentansprüche

1. Für industrielle Anwendungen geeignetes Polypeptid-Polymer-Konjugat, das eine oder mehrere an das Stamm-Polypeptid gekoppelte Polymere aufweist, wobei das Polymer durch die allgemeine Formel
EOₓPO_{y} (I)
wobei x = 1-99%, y = 1-99% und x+y = 100%, **gekennzeichnet** ist.

2. Konjugat nach Anspruch 1, wobei x = 10-90%, y = 10-90% und x+y = 100%.

3. Konjugat nach Anspruch 1 oder 2, wobei das Polymer ein Molekulargewicht von 100 bis 100.000 Da aufweist, insbesondere von 100 bis 50.000 Da aufweist, insbesondere von 100 bis 10.000 Da aufweist.

4. Konjugat nach Anspruch 1 bis 3, wobei das Stamm-Polypeptid ein Molekulargewicht von 1 b is 100 k Da aufweist, insbesondere ein Enzym ein Molekulargewicht von 15 bis 60 kDa ist.

5. Konjugat nach einem der Ansprüche 1 bis 4, wobei das (die) Block- oder Copolymer(e) Ethylenoxideinheiten (EO) und Propylenoxideinheiten (PO) in einem Verhältnis im Bereich von 10:90 oder 20:80 oder 30:70, 40:60, 50:50, 60:40, 70:30, 80:20 oder 90:10 umfasst (umfassen).

6. Konjugat nach einem der Ansprüche 1 bis 5, wobei 1 bis 100 Polymermoleküle, vorzugsweise 4 bis 50, insbesondere 5 bis 35 Polymermoleküle kovalent an das Stamm-Enzym gekoppelt sind.

7. Konjugat nach einem der Ansprüche 1 bis 6, wobei das Polypeptid mikrobiellen Ursprungs wie bakteriellen Ursprungs, vom Ursprung eines Fadenpilzes oder von Ursprung einer Hefe ist.

8. Konjugat nach einem der Ansprüche 1 bis 7, wobei das Polypeptid ein Enzym der Hydrolasegruppe, einschließlich Proteasen, einschließlich Serinprotease, Subtilisine und Metalloproteasen oder Lipasen oder Oxidoreduktasen wie Laccase und Halogenperoxidasen oder Superoxiddismutase, ist.

9. Konjugat nach Anspruch 8, wobei die Stamm-Protease ausgewählt ist aus der Gruppe, einschließend PD498, Savinase®, ProteinaseK, ProteinaseR, Thermitase, Subtilisin DY, Lion Y, Alcalase®, ProteinaseT, Neutrase® und JA16.

10. Konjugat nach Anspruch 9, wobei das Enzym PD498, dargestellt in SEQ ID NR: 1 oder die Protease vom Subtilisin-Typ Subtilisin DY, dargestellt in SEQ ID NR: 3 , oder Lion Y, d argestellt in SEQ ID N R: 4, oder die Metalloprotease Thermolysin, dargestellt in SEQ ID NR: 5, oder Neutrase®, dargestellt in SEQ ID NR: 6, ist.

11. Industrielle Zusammensetzung, umfassend ein Konjugat nach Anspruch 1-10.

12. Industrielle Zusammensetzung nach Anspruch 11, wobei die Zusammensetzung Inhaltsstoffe umfasst, von welchen die Verwendung in Körperpflegezusammensetzungen, insbesondere Hautpflegezusammensetzungen bekannt ist.

13. Industrielle Zusammensetzung nach Anspruch 12, die ein Detergenz wie eine Waschmittelzusammensetzung, Geschirrspülzusammensetzung oder Reinigungszusammensetzung fiir harte Oberflächen ist.

14. Industrielle Zusammensetzung nach Anspruch 11, die eine Zusammensetzung wie ein Nahrungsmittel oder Nahrungsmittelzusatz, insbesondere ein Zusatz zur Brotherstellung oder dergleichen ist.

15. Industrielle Zusammensetzung nach Anspruch 11, die eine Zusammensetzung zur Behandlung von Textilien ist.

16. Verwendung eines Konjugats nach einem der Ansprüche 1-10 zur Reduzierung der Atemallergenität von industriellen Zusammensetzungen nach einem der Ansprüche 11-16.

17. Verfahren zum Reduzieren der Allergenität eines Polypeptids, umfassend an ein Stammpeptid nach einem der Ansprüche 4, 7-10 koppelnde Blockoder Copolymere wie in einem der Ansprüche 1-3, 5, 6 definiert.

## Revendications

1. Un conjugué polymère-peptide convenant à des applications industrielles possédant un ou plusieurs polymères couplés de façon covalente au polypeptide parent, où le polymère est **caractérisé par** la formule générale:
EOₓPO_{y} (I)
où x = 1 à 99%, y = 1 à 99% et x+y = 100%.

2. Le conjugué selon la revendication 1, où x = 10 à 90%, y = 10 à 90% et x+y = 100%.

3. Le conjugué selon les revendications 1 ou 2, où le polymère possède un poids moléculaire allant de 100 à 100 000 Da, en particulier 100 à 50 000 Da, spécialement 100 à 10 000 Da.

4. Le conjugué selon les revendications 1 à 3, où le polypeptide parent possède un poids moléculaire allant de 1 à 100 kDa, en particulier une enzyme, possède un poids moléculaire allant de 15 à 60 kDa.

5. Le conjugué selon l'une quelconque des revendications 1 à 4, où le bloc ou copolymère(s) comprend des unités d'oxyde d'éthylène (EO) et des unités d'oxyde de propylène (PO) dans un ratio compris dans la plage de 10:90 ou 20:80 ou 30:70, 40:60, 50:50, 60:40, 70:30, 80:20 ou 90:10.

6. Le conjugué selon l'une quelconque des revendications 1 à 5, où de 1 à 100 molécules polymères, de préférence 4 à 50, spécialement 5 à 35 molécules polymères, sont couplées de manière covalente à l'enzyme parente.

7. Le conjugué selon l'une quelconque des revendications 1 à 6, où le polypeptide est d'origine microbienne, comme une origine bactérienne, fongique filamenteuse ou de levure.

8. Le conjugué selon l'une quelconque des revendications 1 à 7, où le polypeptide est une enzyme du groupe de l'hydrolase, comprenant les protéases, comprenant les protéases sérines, comme les subtilisines et les métalloprotéases, ou les lipases, ou les oxydoréductases, comme la laccase et les haloperoxydases, ou la dismutase Superoxyde.

9. Le conjugué selon la revendication 8, où la protéase parente est choisie dans le groupe comprenant la PD498, la Savinase®, la ProtéinaseK, la ProtéinaseR, la Thermitase, la Subtilisine DY, la Lion Y, l'Alcalase®, la ProtéinaseT, la Neutrase® et la JA16.

10. Le conjugué selon la revendication 9, où l'enzyme est la PD498 représentée par la SEQ ID NO : 1, ou la protéase de type subtilisine Subtilisine DY représentée par la SEQ ID NO : 3, ou la Lion Y représentée par la SEQ ID NO : 4, ou la métalloprotéase Thermolysine représentée par la SEQ ID NO : 5, ou la Neutrase® représentée par la SEQ ID NO : 6.

11. Une composition industrielle comprenant un conjugué selon la revendication 1 à 10.

12. La composition industrielle selon la revendication 11, où la composition comprend des ingredients connus pour leur utilisation dans les compositions pour soins personnels, en particulier les compositions pour soins de peau.

13. La composition industrielle selon la revendication 12, qui est un détergent, comme une composition de détergent de lessive, un composition pour lave-vaisselle ou une composition de nettoyage de surfaces dures.

14. La composition industrielle selon la revendication 11, qui est une composition, comme un additif alimentaire ou nutritif, spécialement un additif pour fabriquer du pain ou analogue.

15. La composition industrielle selon la revendication 11, qui est une composition de traitement des textiles.

16. L'utilisation d'un conjugué selon l'une quelconque des revendications 1 à 10 pour réduire l'allergénicité des compositions industrielles selon l'une quelconque des revendications 11 à 16.

17. Un précédé pour réduire l'allergénicité d'un polypeptide, comprenant le couplage de blocs ou copolymères tels que définis dans l'une quelconque des revendications 1 à 3, 5, 6 à un polypeptide parent de l'une quelconque des revendications 4, 7 à 10.


### SEQUENCE LISTING

<110> Novo Nordisk A/S
<120> A polypeptide-polymer conjugate
<130> seq
<140>
   <141>
<160> 6
<170> PatentIn Ver. 2.1
<210> 1
   <211> 840
   <212> DNA
   <213> Bacillus sp. PD498
<400> 1
   tggtcaccga atgaccctta ctattctgct taccagtatg gaccacaaaa cacctcaacc 60
   cctgctgcct gggatgtaac ccgtggaagc agcactcaaa cggtggcggt ccttgattcc 120
   ggagtggatt ataaccaccc tgatcttgca agaaaagtaa taaaagggta cgactttatc 180
   gacagggaca ataacccaat ggatcttaac ggacatggta cccatgttgc cggtactgtt 240
   gctgctgata cgaacaatgg aattggcgta gccggtatgg caccagatac gaagatcctt 300
   gccgtacggg tccttgatgc caatggaagt ggctcacttg acagcattgc ctcaggtatc 360
   cgctatgctg ctgatcaagg ggcaaaggta ctcaacctct cccttggttg cgaatgcaac 420
   tccacaactc ttaagagtgc cgtcgactat gcatggaaca aaggagctgt agtcgttgct 480
   gctgcaggga atgacaatgt atcccgtaca ttccaaccag cttcttaccc taatgccatt 540
   gcagtaggtg ccattgactc caatgatcga aaagcatcat tctccaatta cggaacgtgg 600
   gtggatgtca ctgctccagg tgtgaacata gcatcaaccg ttccgaataa tggctactcc 660
   tacatgtctg gtacgtccat ggcatcccct cacgtggccg gtttggctgc tttgttggca 720
   agtcaaggta agaataacgt acaaatccgc caggccattg agcaaaccgc cgataagatc 780
   tctggcactg gaacaaactt caagtatggt aaaatcaact caaacaaagc tgtaagatac 840
<210> 2
   <211> 280
   <212> PRT
   <213> Bacillus sp. PD498
<400> 2
   Trp Ser Pro Asn Asp Pro Tyr Tyr Ser Ala Tyr Gln Tyr Gly Pro Gln
    1               5                  10                  15
   Asn Thr Ser Thr Pro Ala Ala Trp Asp Val Thr Arg Gly Ser Ser Thr
               20                  25                  30
   Gln Thr Val Ala Val Leu Asp Ser Gly Val Asp Tyr Asn His Pro Asp
           35                  40                  45
   Leu Ala Arg Lys Val Ile Lys Gly Tyr Asp Phe Ile Asp Arg Asp Asn
       50                  55                  60
   Asn Pro Met Asp Leu Asn Gly His Gly Thr His Val Ala Gly Thr Val
   65                  70                  75                  80
   Ala Ala Asp Thr Asn Asn Gly Ile Gly Val Ala Gly Met Ala Pro Asp
                   85                  90                  95
   Thr Lys Ile Leu Ala Val Arg Val Leu Asp Ala Asn Gly Ser Gly Ser
               100                 105                 110
   Leu Asp Ser Ile Ala Ser Gly Ile Arg Tyr Ala Ala Asp Gln Gly Ala
           115                 120                 125
   Lys Val Leu Asn Leu Ser Leu Gly Cys Glu Cys Asn Ser Thr Thr Leu
       130                 135                 140
   Lys Ser Ala Val Asp Tyr Ala Trp Asn Lys Gly Ala Val Val Val Ala
   145                 150                 155                 160
   Ala Ala Gly Asn Asp Asn Val Ser Arg Thr Phe Gln Pro Ala Ser Tyr
                   165                 170                 175
   Pro Asn Ala Ile Ala Val Gly Ala Ile Asp Ser Asn Asp Arg Lys Ala
               180                 185                 190
   Ser Phe Ser Asn Tyr Gly Thr Trp Val Asp Val Thr Ala Pro Gly Val
           195                 200                 205
   Asn Ile Ala Ser Thr Val Pro Asn Asn Gly Tyr Ser Tyr Met Ser Gly
       210                 215                 220
   Thr Ser Met Ala Ser Pro His Val Ala Gly Leu Ala Ala Leu Leu Ala
   225                 230                 235                 240
   Ser Gln Gly Lys Asn Asn Val Gln Ile Arg Gln Ala Ile Glu Gln Thr
                   245                 250                 255
   Ala Asp Lys Ile Ser Gly Thr Gly Thr Asn Phe Lys Tyr Gly Lys Ile
               260                 265                 270
   Asn Ser Asn Lys Ala Val Arg Tyr
           275                 280
<210> 3
   <211> 274
   <212> PRT
   <213> Bacillus sp. variant
<400> 3
<210> 4
   <211> 433
   <212> PRT
<213> Bacillus sp. Y
<400> 4
   Asn Asp Val Ala Arg Gly Ile Val Lys Ala Asp Val Ala Gln Asn Asn
   1               5                   10                  15
   Tyr Gly Leu Tyr Gly Gln Gly Gln Leu Val Ala Val Ala Asp Thr Gly
               20                  25                  30
   Leu Asp Thr Gly Arg Asn Asp Ser Ser Met His Glu Ala Phe Arg Gly
           35                  40                  45
   Lys Ile Thr Ala Leu Tyr Ala Leu Gly Arg Thr Asn Asn Ala Ser Asp
       50                  55                  60
   Pro Asn Gly His Gly Thr His Val Ala Gly Ser Val Leu Gly Asn Ala
   65                  70                  75                  80
   Leu Asn Lys Gly Met Ala Pro Gln Ala Asn Leu Val Phe Gln Ser Ile
                   85                  90                  95
   Met Asp Ser Ser Gly Gly Leu Gly Gly Leu Pro Ser Asn Leu Asn Thr
               100                 105                 110
   Leu Phe Ser Gln Ala Trp Asn Ala Gly Ala Arg Ile His Thr Asn Ser
           115                 120                 125
   Trp Gly Ala Pro Val Asn Gly Ala Tyr Thr Ala Asn Ser Arg Gln Val
       130                 135                 140
   Asp Glu Tyr Val Arg Asn Asn Asp Met Thr Val Leu Phe Ala Ala Gly
   145                 150                 155                 160
   Asn Glu Gly Pro Asn Ser Gly Thr Ile Ser Ala Pro Gly Thr Ala Lys
                   165                 170                 175
   Asn Ala Ile Thr Val Gly Ala Thr Glu Asn Tyr Arg Pro Ser Phe Gly
               180                 185                 190
   Ser Ile Ala Asp Asn Pro Asn His Ile Ala Gln Phe Ser Ser Arg Gly
           195                 200                 205
   Ala Thr Arg Asp Gly Arg Ile Lys Pro Asp Val Thr Ala Pro Gly Thr
       210                 215                 220
   Phe Ile Leu Ser Ala Arg Ser Ser Leu Ala Pro Asp Ser Ser Phe Trp
   225                 230                 235                 240
   Ala Asn Tyr Asn Ser Lys Tyr Ala Tyr Met Gly Gly Thr Ser Met Ala
                   245                 250                 255
   Thr Pro Ile Val Ala Gly Asn Val Ala Gln Leu Arg Glu His Phe Ile
               260                 265                 270
   Lys Asn Arg Gly Ile Thr Pro Lys Pro Ser Leu Ile Lys Ala Ala Leu
           275                 280                 285
   Ile Ala Gly Ala Thr Asp Val Gly Leu Gly Tyr Pro Ser Gly Asp Gln
       290                 295                 300
   Gly Trp Gly Arg Val Thr Leu Asp Lys Ser Leu Asn Val Ala Tyr Val
   305                 310                 315                 320
   Asn Glu Ala Thr Ala Leu Ala Thr Gly Gln Lys Ala Thr Tyr Ser Phe
                   325                 330                 335
   Gln Ala Gln Ala Gly Lys Pro Leu Lys Ile Ser Leu Val Trp Thr Asp
               340                 345                 350
   Ala Pro Gly Ser Thr Thr Ala Ser Tyr Thr Leu Val Asn Asp Leu Asp
           355                 360                 365
   Leu Val Ile Thr Ala Pro Asn Gly Gln Lys Tyr Val Gly Asn Asp Phe
       370                 375                 380
   Ser Tyr Pro Tyr Asp Asn Asn Trp Asp Gly Arg Asn Asn Val Glu Asn
   385                 390                 395                 400
   Val Phe Ile Asn Ala Pro Gln Ser Gly Thr Tyr Ile Ile Glu Val Gln
                   405                 410                 415
   Ala Tyr Asn Val Pro Ser Gly Pro Gln Arg Phe Ser Leu Ala Ile Val
               420                 425                 430
   His
<210> 5
   <211> 316
   <212> PRT
   <213> Bacillus thermoproteolyticus
<400> 5
<210> 6
   <211> 300
   <212> PRT
   <213> Bacillus amyloliquefaciens
<400> 6
   Ala Ala Thr Thr Gly Thr Gly Thr Thr Leu Lys Gly Lys Thr Val Ser
    1               5                  10                  15
   Leu Asn Ile Ser Ser Glu Ser Gly Lys Tyr Val Leu Arg Asp Leu Ser
               20                  25                  30
   Lys Pro Thr Gly Thr Gln Ile Ile Thr Thr Asp Leu Gln Asn Arg Glu
           35                  40                  45
   Tyr Asn Leu Pro Gly Thr Leu Val Ser Ser Thr Thr Asn Gln Phe Thr
       50                  55                  60
   Thr Ser Ser Gln Arg Ala Ala Val Asp Ala His Tyr Asn Leu Gly Lys
   65                  70                  75                  80
   Val Tyr Asp Tyr Phe Tyr Gln Lys Phe Asn Arg Asn Ser Tyr Asp Asn
                   85                  90                  95
   Lys Gly Gly Lys Ile Val Ser Ser Val His Tyr Gly Ser Arg Tyr Asn
               100                 105                 110
   Asn Ala Ala Trp Ile Gly Asp Gln Met Ile Tyr Gly Asp Gly Asp Gly
           115                 120                 125
   Ser Phe Phe Ser Pro Leu Ser Gly Ser Met Asp Val Thr Ala His Glu
       130                 135                 140
   Met Thr His Gly Val Thr Gln Glu Thr Ala Asn Leu Asn Tyr Glu Asn
   145                 150                 155                 160
   Gln Pro Gly Ala Leu Asn Glu Ser Phe Ser Asp Val Phe Gly Tyr Phe
                   165                 170                 175
   Asn Asp Thr Glu Asp Trp Asp Ile Gly Glu Asp Ile Thr Val Ser Gln
               180                 185                 190
   Pro Ala Leu Arg Ser Leu Ser Asn Pro Thr Lys Tyr Gly Gln Pro Asp
           195                 200                 205
   Asn Phe Lys Asn Tyr Lys Asn Leu Pro Asn Thr Asp Ala Ala Ala Thr
       210                 215                 220
   Thr Gly Thr Gly Tyr Tyr Leu Lys Gly Lys Thr Val Ser Leu Asn Ile
   225                 230                 235                 240
   Ser Ser Glu Ser Gly Lys Tyr Val Leu Arg Asp Leu Ser Lys Pro Thr
                   245                 250                 255
   Gly Thr Gln Ile Ile Thr Tyr Asp Leu Gln Asn Arg Glu Tyr Asn Leu
               260                 265                 270
   Pro Gly Thr Leu Val Ser Ser Thr Thr Asn Gln Phe Thr Thr Ser Ser
           275                 280                 285
   Gln Arg Ala Ala Val Asp Ala His Tyr Asn Leu Gly
       290                 295                 300